(19) Europäisches Patentamt
European Patent Office
Office européen des brevets

(11) Publication number: **0 154 272 B1**

(12) **EUROPEAN PATENT SPECIFICATION**

(45) Date of publication of patent specification: **08.01.92**

(51) Int. Cl.$^5$: **C12N 15/58**

(21) Application number: **85102031.3**

(22) Date of filing: **23.02.85**

(54) **Production of human urokinase.**

(30) Priority: **27.02.84 JP 37119/84**
**31.01.85 JP 17969/85**

(43) Date of publication of application:
**11.09.85 Bulletin 85/37**

(45) Publication of the grant of the patent:
**08.01.92 Bulletin 92/02**

(84) Designated Contracting States:
**BE DE FR GB NL SE**

(56) References cited:
**EP-A- 0 037 687**
**EP-A- 0 092 182**
**EP-A- 0 093 619**

(73) Proprietor: **GREEN CROSS CORPORATION**
**3-3, Imabashi 1-chome Chuo-ku**
**Osaka(JP)**

(72) Inventor: **Hiramatsu, Ryuji**
**14-1-1008, Kayashimashinwa-cho**
**Neyagawa-shi Osaka(JP)**
Inventor: **Kaneda, Teruo**
**5-51-C-105, Nagisanishi 1-chome**
**Hirakata-shi Osaka(JP)**
Inventor: **Nagai, Masanori**
**10-10, Nishiyamawaki**
**Yahata-shi Kyoto(JP)**
Inventor: **Arimura, Hirofumi**
**18-1-401, Uenosaka 2-chome**
**Toyonaka-shi Osaka(JP)**
Inventor: **Nishida, Masayuki**
**12-1, Kayogaoka 2-chome**
**Nagaokakyo-shi Kyoto(JP)**
Inventor: **Suyama, Tadakazu**
**3-7, Matsuigaoka 4-chome Tanabe-cho**
**Tsuzuki-gun Kyoto(JP)**

(74) Representative: **von Kreisler, Alek, Dipl.-Chem. et al**
**Deichmannhaus am Hauptbahnhof**
**W-5000 Köln 1(DE)**

**EP 0 154 272 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

Field of the Invention

This invention relates to a method of producing glycosylated single-chain pro-urokinase (hereinafter referred to as pro-UK). More particularly, it relates to a method of producing glycosylated pro-UK which comprises recovering mRNA from an established human kidney-derived cell line, preparing cDNA based on said mRNA, inserting the cDNA into a vector, introducing the resulting plasmid into an animal cell to thereby produce a transformant, and recovering glycosylated pro-UK from said animal cell.

Description of the Prior Art

Urokinase is a plasminogen activator obtainable from human urine and can produce excellent therapeutic effects in the treatment of various forms of thrombosis or embolic diseases as well as in the combined use with an anticancer agents. Currently, urokinase is produced mainly from human urine by some or other purification method (cf. Williams, J. R. B.: Br. J. Exp. Pathol., 32, 530).

As a result of cell culture technology development in search of a substitute method, the method comprising isolating human kidney cells, subculturing the cells and recovering urokinase from the culture is becoming a second important method. However, recent improvements in the recombinant DNA technology have made it possible to produce desired proteins on a commercial scale by using microorganisms such as Escherichia coli, Saccharomyces cerevisiae and Bacillus subtilis or by using cultured mammalian cells [cf. Goeddel, D. V., Itakura, K., et al.: Proc. Natl. Acad. Sci. U.S.A., 76, 106 (1979) and Nagata, S., Taira, S. H. and Wissmarn, C.: Nature, 284, 1316 (1980)].

SUMMARY OF THE INVENTION

Under these circumstances, the present inventors studied in an attempt to develop a method of producing urokinase using recombinant DNA techniques. As a result, they obtained mRNA from an established human kidney-derived cell line, prepared the cDNA sequence shown in the following sequence (I) by using said mRNA as the template, cultivated animal cells transformed with a plasmid with said cDNA sequence inserted therein and obtained glycosylated pro-UK, and have now completed the present invention.

Thus, the present invention relates to a method of producing glycosylated singly-chain pro-urokinase which comprises recovering mRNA from an establised human kidney-derived cell line, preparing cDNA based on said mRNA, inserting the cDNA into a vector, introducing the resulting plasmid into an animal cell to thereby produce a transformant, and recovering glycosylated pro-UK from said animal cell.

Sequence(I): the base sequence of the structural gene for pro-UK

```
 1                                      10                                      20
Ser-Asn-Glu-Leu-His-Gln-Val-Pro-Ser-Asn-Cys-Asp-Cys-Leu-Asn-Gly-Gly-Thr-Cys-Val-
AGC AAT GAA CTT CAT CAA GTT CCA TCG AAC TGT GAC TGT CTA AAT GGA GGA ACA TGT GTG

 21                                     30                                      40
Ser-Asn-Lys-Tyr-Phe-Ser-Asn-Ile-His-Trp-Cys-Asn-Cys-Pro-Lys-Lys-Phe-Gly-Gly-Gln-
TCC AAC AAG TAC TTC TCC AAC ATT CAC TGG TGC AAC TGC CCA AAG AAA TTC GGA GGG CAG

 41                                     50                                      60
His-Cys-Glu-Ile-Asp-Lys-Ser-Lys-Thr-Cys-Tyr-Glu-Gly-Asn-Gly-His-Phe-Tyr-Arg-Gly-
CAC TGT GAA ATA GAT AAG TCA AAA ACC TGC TAT GAG GGG AAT GGT CAC TTT TAC CGA GGA

 61                                     70                                      80
Lys-Ala-Ser-Thr-Asp-Thr-Met-Gly-Arg-Pro-Cys-Leu-Pro-Trp-Asn-Ser-Ala-Thr-Val-Leu-
AAG GCC AGC ACT GAC ACC ATG GGC CGG CCC TGC CTG CCC TGG AAC TCT GCC ACT GTC CTT

 81                                     90                                     100
Gln-Gln-Thr-Tyr-His-Ala-His-Arg-Ser-Asp-Ala-Leu-Gln-Leu-Gly-Leu-Gly-Lys-His-Asn-
CAG CAA ACG TAC CAT GCC CAC AGA TCT GAT GCT CTT CAG CTG GGC CTG GGG AAA CAT AAT

101                                    110                                     120
Tyr-Cys-Arg-Asn-Pro-Asp-Asn-Arg-Arg-Arg-Pro-Trp-Cys-Tyr-Val-Gln-Val-Gly-Leu-Lys-
TAC TGC AGG AAC CCA GAC AAC CGG AGG CGA CCC TGG TGC TAT GTG CAG GTG GGC CTA AAG

121                                    130                                     140
Pro-Leu-Val-Gln-Glu-Cys-Met-Val-His-Asp-Cys-Ala-Asp-Gly-Lys-Lys-Pro-Ser-Ser-Pro-
CCG CTT GTC CAA GAG TGC ATG GTG CAT GAC TGC GCA GAT GGA AAA AAG CCC TCC TCT CCT

141                                    150                                     160
Pro-Glu-Glu-Leu-Lys-Phe-Gln-Cys-Gly-Gln-Lys-Thr-Leu-Arg-Pro-Arg-Phe-Lys-Ile-Ile-
CCA GAA GAA TTA AAA TTT CAG TGT GGC CAA AAG ACT CTG AGG CCC CGC TTT AAG ATT ATT
```

(continued on the following page)

```
161                                             170                                                  180
Gly-Gly-Glu-Phe-Thr-Thr-Ile-Glu-Asn-Gln-Pro-Trp-Phe-Ala-Ala-Ile-Tyr-Arg-Arg-His-
GGG GGA GAA TTC ACC ACC ATC GAG AAC CAG CCC TGG TTT GCG GCC ATC TAC AGG AGG CAC

181                                             190                                                  200
Arg-Gly-Gly-Ser-Val-Thr-Tyr-Val-Cys-Gly-Gly-Ser-Leu-Ile-Ser-Pro-Cys-Trp-Val-Ile-
CGG GGG GGC TCT GTC ACC TAC GTG TGT GGA GGC AGC CTC ATC AGC CCT TGC TGG GTG ATC

201                                             210                                                  220
Ser-Ala-Thr-His-Cys-Phe-Ile-Asp-Tyr-Pro-Lys-Lys-Glu-Asp-Tyr-Ile-Val-Tyr-Leu-Gly-
AGC GCC ACA CAC TGC TTC ATT GAT TAC CCA AAG AAG GAG GAC TAC ATC GTC TAC CTG GGT

221                                             230                                                  240
Arg-Ser-Arg-Leu-Asn-Ser-Asn-Thr-Gln-Gly-Glu-Met-Lys-Phe-Glu-Val-Glu-Asn-Leu-Ile-
CGC TCA AGG CTT AAC TCC AAC ACG CAA GGG GAG ATG AAG TTT GAG GTG GAA AAC CTC ATC

241                                             250                                                  260
Leu-His-Lys-Asp-Tyr-Ser-Ala-Asp-Thr-Leu-Ala-His-His-Asn-Asp-Ile-Ala-Leu-Leu-Lys-
CTA CAC AAG GAC TAC AGC GCT GAC ACG CTT GCT CAC CAC AAC GAC ATT GCC TTG CTG AAG

261                                             270                                                  280
Ile-Arg-Ser-Lys-Glu-Gly-Arg-Cys-Ala-Gln-Pro-Ser-Arg-Thr-Ile-Gln-Thr-Ile-Cys-Leu-
ATC CGT TCC AAG GAG GGC AGG TGT GCG CAG CCA TCC CGG ACT ATA CAG ACC ATC TGC CTG

281                                             290                                                  300
Pro-Ser-Met-Tyr-Asn-Asp-Pro-Gln-Phe-Gly-Thr-Ser-Cys-Glu-Ile-Thr-Gly-Phe-Gly-Lys-
CCC TCG ATG TAT AAC GAT CCC CAG TTT GGC ACA AGC TGT GAG ATC ACT GGC TTT GGA AAA

301                                             310                                                  320
Glu-Asn-Ser-Thr-Asp-Tyr-Leu-Tyr-Pro-Glu-Gln-Leu-Lys-Met-Thr-Val-Val-Lys-Leu-Ile-
GAG AAT TCT ACC GAC TAT CTC TAT CCG GAG CAG CTG AAG ATG ACT GTT GTG AAG CTG ATT

321                                             330                                                  340
Ser-His-Arg-Glu-Cys-Gln-Gln-Pro-His-Tyr-Tyr-Gly-Ser-Glu-Val-Thr-Thr-Lys-Met-Leu-
TCC CAC CGG GAG TGT CAG CAG CCC CAC TAC TAC GGC TCT GAA GTC ACC ACC AAA ATG CTG
```

(continued on the following page)

```
341                                         350
Cys-Ala-Ala-Asp-Pro-Gln-Trp-Lys-Thr-Asp-Ser-Cys-Gln-Gly-Asp-Ser-Gly-Gly-Pro-Leu-
TGT GCT GCT GAC CCA CAG TGG AAA ACA GAT TCC TGC CAG GGA GAC TCA GGG GGA CCC CTC
                                                                            360
361                                         370
Val-Cys-Ser-Leu-Gln-Gly-Arg-Met-Thr-Leu-Thr-Gly-Ile-Val-Ser-Trp-Gly-Arg-Gly-Cys-
GTC TGT TCC CTC CAA GGC CGC ATG ACT TTG ACT GGA ATT GTG AGC TGG GGC CGT GGA TGT
                                                                            380
381                                         390
Ala-Leu-Lys-Asp-Lys-Pro-Gly-Val-Tyr-Thr-Arg-Val-Ser-His-Phe-Leu-Pro-Trp-Ile-Arg-
GCC CTG AAG GAC AAG CCA GGC GTC TAC ACG AGA GTC TCA CAC TTC TTA CCC TGG ATC CGC
                                                                            400
401                                         410
Ser-His-Thr-Lys-Glu-Glu-Asn-Gly-Leu-Ala-Leu
AGT CAC ACC AAG GAA GAG AAT GGC CTG GCC CTC
```

The pro-UK according to the invention was produced in the following manner:

Using a mRNA fraction (185 to 20S) containing urokinase mRNA as isolated and purified from an established, human kidney-derived cell line, cDNA was synthesized. Starting with 20 µg of mRNA and using reverse transcriptase and DNA polymerase I, there was obtained 3.3 µg of double-stranded cDNA (ds cDNA). After removal of the single-stranded portion by treatment with SI nuclease, the double stranded cDNA was subjected to sucrose density gradient centrifugation to thereby collect a large molecular ds cDNA fraction (750 ng). A 1/5 portion (150 ng) of this double-stranded cDNA was subjected to treatment for

adding a poly(C) tail. A poly(G) tail was added to the Escherichia coli plasmid pBR322 prepared separately. Both the addition products were joined together by annealing. Escherichia coli was transformed with the pBR322 plasmid containing the ds cDNA inserted therein, giving about 70,000 transformants, over 90% of which showed ampicillin resistance indicative of the insertion of the cDNA at the PstI site of pBR322.

Of these transformants, 10,000 colonies were subjected to screening for selecting urokinase cDNA. Based on the amino acid sequence of urokinase, a mixture of eight 14-mer oligodeoxyribonucleotides, namely

$$NH_2\text{ -Glu-Met-Lys-Phe-Glu-COOH}$$

$$3'\text{ -CT}^{T}_{C}\text{-TAC-TT}^{T}_{C}\text{- AA}^{G}_{A}\text{-CT-5'}$$

was synthetically prepared and used as a hybridization probe in screening for urokinase cDNA by colony hybridization. As a result, four positive colonies expected to contain urokinase cDNA were obtained. Plasmid DNAs (pUK1 through 4) were prepared from these four colonies and measured for the size of cDNA. pUK1, pUK2, pUK3 and pUK4 were found to contain 1900 bp, 170 bp, 1150 bp and 1300 bp cDNAs, respectively. Detailed restriction enzyme cleavage maps were prepared for pUK1, pUK2 and pUK3, whereby these cDNAs gave restriction maps overlapping one another (cf. Fig. 1). The partial DNA base sequence each of pUK1 and pUK4 as determined by the Maxam-Gilbert method was in agreement with the DNA base sequence deduced from the known amino acid sequence of urokinase. pUK1 had about 670 bp-long 3'-noncoding region-containing cDNA whereas pUK4 had a signal peptide and 5'-noncoding region-containing cDNA. These pUK1 and pUK4 were joined together taking advantage of the BglII site to thereby construct a plasmid containing the entire translation region for urokinase. Using this plasmid, the DNA base sequence of the entire urokinase translation region and its neighborhood was determined and, based on said sequence, the amino acid sequence of the signal peptide and translation region was further determined [hereinafter reffered to as sequence (II)].

Sequence (II): the base sequence of the signal sequence and noncoding region of the pro-UK structural gene and the corresponding amino acid sequence

```
5' TCCACCTGTCCCCGCAGCGCCGGCTCGCGCCCTCCTGCCGCAGCCACCGAGCCGCCGTCTAGCGCCCCGACCTCGCCACC

-20                                     -10                                     -1
Met Arg Ala Leu Leu Ala Arg Leu Leu Leu Cys Val Leu Val Val Ser Asp Ser Lys Gly
ATG AGA GCC CTG CTG GCG CGC CTG CTT CTC TGC GTC CTG GTC GTG AGC GAC TCC AAA GGC

 1                                   10                                      20
Ser-Asn-Glu-Leu-His-Gln-Val-Pro-Ser-Asn-Cys-Asp-Cys-Leu-Asn-Gly-Gly-Thr-Cys-Val-
AGC AAT GAA CTT CAT CAA GTT CCA TCG AAC TGT GAC TGT CTA AAT GGA GGA ACA TGT GTG

21                                   30                                      40
Ser-Asn-Lys-Tyr-Phe-Ser-Asn-Ile-His-Trp-Cys-Asn-Cys-Pro-Lys-Lys-Phe-Gly-Gly-Gln-
TCC AAC AAG TAC TTC TCC AAC ATT CAC TGG TGC AAC TGC CCA AAG AAA TTC GGA GGG CAG

41                                   50                                      60
His-Cys-Glu-Ile-Asp-Lys-Ser-Lys-Thr-Cys-Tyr-Glu-Gly-Asn-Gly-His-Phe-Tyr-Arg-Gly-
CAC TGT GAA ATA GAT AAG TCA AAA ACC TGC TAT GAG GGG AAT GGT CAC TTT TAC CGA GGA

61                                   70                                      80
Lys-Ala-Ser-Thr-Asp-Thr-Met-Gly-Arg-Pro-Cys-Leu-Pro-Trp-Asn-Ser-Ala-Thr-Val-Leu-
AAG GCC AGC ACT GAC ACC ATG GGC CGG CCC TGC CTG CCC TGG AAC TCT GCC ACT GTC CTT

81                                   90                                     100
Gln-Gln-Thr-Tyr-His-Ala-His-Arg-Ser-Asp-Ala-Leu-Gln-Leu-Gly-Leu-Gly-Lys-His-Asn-
CAG CAA ACG TAC CAT GCC CAC AGA TCT GAT GCT CTT CAG CTG GGC CTG GGG AAA CAT AAT

101                                 110                                     120
Tyr-Cys-Arg-Asn-Pro-Asp-Asn-Arg-Arg-Arg-Pro-Trp-Cys-Tyr-Val-Gln-Val-Gly-Leu-Lys-
TAC TGC AGG AAC CCA GAC AAC CGG AGG CGA CCC TGG TGC TAT GTG CAG GTG GGC CTA AAG
```

(continued on the following page)

```
121                                                         130                                                         140
Pro-Leu-Val-Gln-Glu-Cys-Met-Val-His-Asp-Cys-Ala-Asp-Gly-Lys-Lys-Pro-Ser-Ser-Pro-
CCG CTT GTC CAA GAG TGC ATG GTG CAT GAC TGC GCA GAT GGA AAA AAG CCC TCC TCT CCT

141                                                         150                                                         160
Pro-Glu-Glu-Leu-Lys-Phe-Gln-Cys-Gly-Gln-Lys-Thr-Leu-Arg-Pro-Arg-Phe-Lys-Ile-Ile-
CCA GAA GAA TTA AAA TTT CAG TGT GGC CAA AAG ACT CTG AGG CCC CGC TTT AAG ATT ATT

161                                                         170                                                         180
Gly-Gly-Glu-Phe-Thr-Thr-Ile-Glu-Asn-Gln-Pro-Trp-Phe-Ala-Ala-Ile-Tyr-Arg-Arg-His-
GGG GGA GAA TTC ACC ACC ATC GAG AAC CAG CCC TGG TTT GCG GCC ATC TAC AGG AGG CAC

181                                                         190                                                         200
Arg-Gly-Gly-Ser-Val-Thr-Tyr-Val-Cys-Gly-Gly-Ser-Leu-Ile-Ser-Pro-Cys-Trp-Val-Ile-
CGG GGG GGC TCT GTC ACC TAC GTG TGT GGA GGC AGC CTC ATC AGC CCT TGC TGG GTG ATC

201                                                         210                                                         220
Ser-Ala-Thr-His-Cys-Phe-Ile-Asp-Tyr-Pro-Lys-Lys-Glu-Asp-Tyr-Ile-Val-Tyr-Leu-Gly-
AGC GCC ACA CAC TGC TTC ATT GAT TAC CCA AAG AAG GAG GAC TAC ATC GTC TAC CTG GGT

221                                                         230                                                         240
Arg-Ser-Arg-Leu-Asn-Ser-Asn-Thr-Gln-Gly-Glu-Met-Lys-Phe-Glu-Val-Glu-Asn-Leu-Ile-
CGC TCA AGG CTT AAC TCC AAC ACG CAA GGG GAG ATG AAG TTT GAG GTG GAA AAC CTC ATC

241                                                         250                                                         260
Leu-His-Lys-Asp-Tyr-Ser-Ala-Asp-Thr-Leu-Ala-His-His-Asn-Asp-Ile-Ala-Leu-Leu-Lys-
CTA CAC AAG GAC TAC AGC GCT GAC ACG CTT GCT CAC CAC AAC GAC ATT GCC TTG CTG AAG

261                                                         270                                                         280
Ile-Arg-Ser-Lys-Glu-Gly-Arg-Cys-Ala-Gln-Pro-Ser-Arg-Thr-Ile-Gln-Thr-Ile-Cys-Leu-
ATC CGT TCC AAG GAG GGC AGG TGT GCG CAG CCA TCC CGG ACT ATA CAG ACC ATC TGC CTG
```

(continued on the following page)

```
                                                   290                                                                300
281
Pro-Ser-Met-Tyr-Asn-Asp-Pro-Gln-Phe-Gly-Thr-Ser-Cys-Glu-Ile-Thr-Gly-Phe-Gly-Lys-
CCC TCG ATG TAT AAC GAT CCC CAG TTT GGC ACA AGC TGT GAG ATC ACT GGC TTT GGA AAA

                                                   310                                                                320
301
Glu-Asn-Ser-Thr-Asp-Tyr-Leu-Tyr-Pro-Glu-Gln-Leu-Lys-Met-Thr-Val-Val-Lys-Leu-Ile-
GAG AAT TCT ACC GAC TAT CTC TAT CCG GAG CAG CTG AAG ATG ACT GTT GTG AAG CTG ATT

                                                   330                                                                340
321
Ser-His-Arg-Glu-Cys-Gln-Gln-Pro-His-Tyr-Tyr-Gly-Ser-Glu-Val-Thr-Thr-Lys-Met-Leu-
TCC CAC CGG GAG TGT CAG CAG CCC CAC TAC TAC GGC TCT GAA GTC ACC ACC AAA ATG CTG

                                                   350                                                                360
341
Cys-Ala-Ala-Asp-Pro-Gln-Trp-Lys-Thr-Asp-Ser-Cys-Gln-Gly-Asp-Ser-Gly-Gly-Pro-Leu-
TGT GCT GCT GAC CCA CAG TGG AAA ACA GAT TCC TGC CAG GGA GAC TCA GGG GGA CCC CTC

                                                   370                                                                380
361
Val-Cys-Ser-Leu-Gln-Gly-Arg-Met-Thr-Leu-Thr-Gly-Ile-Val-Ser-Trp-Gly-Arg-Gly-Cys-
GTC TGT TCC CTC CAA GGC CGC ATG ACT TTG ACT GGA ATT GTG AGC TGG GGC CGT GGA TGT

                                                   390                                                                400
381
Ala-Leu-Lys-Asp-Lys-Pro-Gly-Val-Tyr-Thr-Arg-Val-Ser-His-Phe-Leu-Pro-Trp-Ile-Arg-
GCC CTG AAG GAC AAG CCA GGC GTC TAC ACG AGA GTC TCA CAC TTC TTA CCC TGG ATC CGC

                                                   410
401
Ser-His-Thr-Lys-Glu-Glu-Asn-Gly-Leu-Ala-Leu Stop
AGT CAC ACC AAG GAA GAG AAT GGC CTG GCC CTC TGA GGGTCCCCAGGGAGGAAACGGGCACCACCCGC

TTTCTTGCTGGTTGTCATTTTTGCAGTAGAGTCATCTCCATCAGCTGTAAGAAGAGACTGGGAAGA .....3'
```

It was thus revealed that urokinase is constituted by a signal peptide composed of 20 amino acids and the subsequent 411 amino acids. Furthermore, the DNA base sequence of urokinase cDNA and other data revealed that a 5'-noncoding region comprising not less than 80 bases and a 3'-noncoding region comprising not less than 800 bases occur in urokinase mRNA [cf. Sequence (II)].

The thus-obtained urokinase cDNA was inserted into a vector usable in a heterologous gene expression system in animal cells, followed by introduction into animal cells. Production of glycosylated pro-UK with molecular weight of 54,000 dalton in the transformant cells thus obtained was confirmed.

BRIEF DESCRIPTION OF THE DRAWING

In the accompanying drawings,

Fig. 1 shows the restriction enzyme map of cDNA;

Fig. 2 shows the ultracentrifugation sedimentation pattern of cDNA;

Fig. 3 shows the structure of pro-UK mRNA and the position of the synthetic DNA probe;

Fig. 4 shows the restriction enzyme map each of pUK1, pUK4 and pUK18, pUK1 having cDNA of about 1,900 bp, pUK4 having cDNA of about 1,300 bp and pUK18 having cDNA of about 1,450 bp, their restriction enzyme maps overlapping with one another;

Fig. 5 shows the restriction enzyme map of cDNA, wherein the restriction enzyme of cDNA is given in a unified form on the basis of Fig. 7; *, X and O indicating the position of [$^{32}$P] labelling and the arrow indicating the direction of sequencing; *, X and O meaning that the sequencing was performed using pUK1, pUK4 and pUK18, respectively;

Fig. 6 shows the flowchart for the construction of plasmid pUK33 by ligation of pUK1 with pUK4, wherein E, H and B stand for EcoRI, HindIII and BamHI, respectively;

Fig. 7 shows the flowchart for the ligation of pcDV1 with pL1;

Fig. 8 shows the restriction enzyme map of pSVI-GI;

Fig. 9 shows the restriction enzyme map of pSV-GI-preUK; and pSV-GI-NEO;

Fig. 10 shows the structure of preUK cDNA and its neighborhood in pSV-GI-preUk, wherein NC, SS and SJ stand for noncoding region, signal sequence and splicing junction, respectively;

Fig. 11 shows the results in SDS-PAGE for measurement of the molecular weight of urokinase-like protein in the culture supernatant.

| | |
|---|---|
| lane 1,6 | : Urinary urokinase |
| lane 2 | : Culture supernatant of human kidney-derived cell line. (54k in molecular weight band was detected.) |
| lane 3,4,5 | : Culture supernatant of CHD-$K_1$, C-68 and C-61 cells. (54k, 34k and 20k in molecular weight bands were detected.) |

DETAILED DESCRIPTION OF THE INVENTION

(1) Isolation and purification of mRNA

(a) Cells used

For extraction of whole RNA from human kidney-derived established cell line capable of producing urokinase, these cells were first allowed to form a monolayer in a multistage plastic incubator and a Roux flask using a growth medium. Thereafter, the medium was changed for a maintenance medium and the cultivation was continued for further 10-15 hours. 2 to 5 x $10^9$ cells at that stage were collected by trypsin treatment, followed by whole RNA extraction.

(b) Recovery of mRNA

Using the method. disclosed in Japanese Tokkyo Kokai Koho 58-148899 (filed by Green Cross Corp.), mRNA having a size of 19-205 with which urokinase activity was noted upon injection into Xenopus laevis oocytes, was isolated and purified.

(2) Synthesis of single-stranded cDNA

The synthesis of cDNA was performed by the method of Maruin, P. W. et al.: J. Biol. Chem., 253, 2483-2495 (1978).

(a) To the reaction mixture specified in Table 1, there was added 20 $\mu$g of urokinase mRNA heated at 65° C for 5 minutes and rapidly cooled on ice. After stirring well in an Eppendorff tube, the mixture was allowed to stand on ice.

Table 1

| Reaction mixture | Amount | Final concentration |
|---|---|---|
| 0.5M Tris-HCl (pH8.3) | 20 μl | 50mM |
| 1.4M KCl | 10 μl | 70mM |
| 0.25M $MgCl_2$ | 8 μl | 10mM |
| 0.05M dNTP | Each 2 μl | Each 0.5mM |
| 50μCi α-$^{32}$P-dCTP | 10 μl | |
| 0.2M DTT | 10 μl | 10mM |
| Oligo $(dt)_{12-18}$ (250 μg/ml) | 20 μl | 25 μg/ml |
| mRNA | 10 or 20 μg | |
| AMV reverse transcriptase | 160 units | 800 u/ml |
| RNase inhibitor | 22 units | 70 u/ml |
| $H_2O$ | To make the Total 200 μl | |

(b) Single-stranded cDNA was synthesized by reaction at 46°C for 10-12 minutes.
(c) The reaction was terminated by adding 20 μl of 0.5 M EDTA to the reaction mixture.
(d) After extraction with 200 μl of phenol-chloroform, the mixture was centrifuged at 10,000 rpm for 3 minutes. The aqueous layer was separated. The lower phenol layer was reextracted with an equal amount of Sephadex G-100 buffer (10 mM Tris-HCl pH 8.0, 1 mM EDTA, 100 mM NaCl). Both the aqueous layers were combined and 60 μl of 80% glycerin was added to the resulting reaction mixture.
(e) The reaction mixture was applied to a Sephadex G-100 column, followed by elution with G-100 buffer. The eluate was fractionated by 5 drops.
(f) Each fraction was tested by means of a Cherenkov counter and radioactive peak portions were collected.
(g) Thereto were added 1/10 volume of 3 M sodium acetate buffer, 10 μg of tRNA and 2 volumes of ethanol, and the mixture was cooled at -80°C for 20 minutes.
(h) The precipitate was collected by centrifugation (15,000 rpm, 10 minutes) and dried under reduced pressure.
(i) The precipitate was dissolved in 300 μl of 0.1 N aqueous NaOH, followed by incubation at 70°C for 20 minutes and cooling with water.
(j) The solution was neutralized by adding about 30 μl of 1 N HCl. Thereafter, a 2 μl portion was sampled and measured for radioactivity.
(k) 10 μg of tRAN was added, followed by precipitation with ethanol. The precipitate was collected by centrifugation and dried under reduced pressure.
(l) The synthesis of single-stranded cDNA was thus completed. The yield of single-stranded cDNA was determined by recovering single-stranded cDNA-mRNA hybrid by G-100 column chromatography and degradative removal of mRNA by hot alkali treatment. As a result, 3.65 μg of urokinase-encoding single-stranded cDNA was obtained.

(3) Synthesis of second strand DNA

For the synthesis of second strand DNA, DNA was first synthesized using DNA polymerase I and then the DNA chain was extended using reverse transcriptase.

(a) 100 μl of single-stranded cDNA was suspended in 2 x reaction mixture (Table 2).

Table 2

| Reaction mixture | Amount | Concentration |
|---|---|---|
| 0.5 M HEPES-NaOH (pH6.9) | 20 μl (combined) | 100 mM |
| 67 mM $MgCl_2$ | | 13.4 mM |
| 0.7 M KCl | | 140 mM |
| 0.2 M DTT | 5 μl | 10 mM |
| 5 mM dXTP | 20 μl | 1 mM |
| $H_2O$ | To make the total 100 μl | |

(b) Incubation was conducted at 60°C for 2 minutes, followed by centrifugation (15,000 rpm, 2.5 minutes). The supernatant was collected.

(c) Following addition of 50 μl of water to the precipitate, the procedure of (b) was repeated. Both the supernatants were combined.

(d) 50 units of DNA polymerase I Klenow fragment was added and the total amount was made 200 μl.

(e) Incubation was performed at 15°C overnight.

(f) The reaction was terminated by adding 20 μl of 0.2 M EDTA (pH 8.0), then an equal volume (220 μl) of phenol-chloroform solution was added and the mixture was stirred and centrifuged. The aqueous layer was collected. The phenol layer was reextracted with 200 μl of TE buffer (10 mM Tris-HCl pH 8.0, 1 mM EDTA).

(g) Both the aqueous layers were combined and 60 μl of 80% glycerin was added.

(h) The resulting mixture was applied to a Sephadex G-100 column, followed by elution with G-100 buffer. The eluate was fractionated by 5 drops.

(i) Each fraction was tested by means of a Cherenkov counter, and radioactivity peak fractions were collected. 20 μl aliquot was mixed with 10 ml of ACS II and the mixture was used for radioactivity measurement. To the remaining portion of the liquid, there were added 5 μg of tRNA and 1/10 volumn of 3 M sodium acetate buffer, followed by precipitation with ethanol.

(j) The precipitate was collected by centrifugation (15,000 rpm, 10 minutes) and dried under reduced pressure.

There was thus obtained 5.3 μg of ds cDNA.

Thereafter, synthetic extention of second strand DNA was conducted using reverse transcriptase.

(4) Synthetic extention of second strand DNA using reverse transcriptase

(a) The ds cDNA obtained was dissolved in 20 μl of water. To the solution was added 30 μl of the reaction mixture given in Table 3, followed by stirring.

Table 3

| Reaction mixture | Amount |
|---|---|
| 1 M Tris-HCl (pH 8.3) | 5 µl |
| 1M KCl | 7 µl |
| 250 mM $MgCl_2$ | 2 µl |
| dNTP (20 mM) | 2.5 µl |
| 700 mM β-mercaptoethanol | 2 µl |
| AMV reverse transcriptase | 2 µl |
| $H_2O$ | To make the total 30 µl |

(b) This mixture was incubated at 42° C for 60 minutes.
(c) The reaction was terminated by adding 5 µl of 0.2 M EDTA.
(d) Deproteinization was performed by adding an equal volume (55 µl) of phenol-chloroform.
(e) Thereafter, the same procedures [(g)-(j)] as in the synthesis of ds cDNA using polymerase I were performed, followed by digestion treatment of the single-stranded DNA portion with SI nuclease according to Shenk, T. E. et al. [cf. Proc. Natl. Acad. Sci. U.S.A., 72, 989 (1975)], as mentioned below.

(5) Digestion of single-stranded DNA portion with SI nuclease

(a) The ds cDNA was suspended in 100 µl of buffer for SI nuclease [0.3 M NaCl, 30 mM sodium acetate (pH 4.5), 3 mM $ZnCl_2$] and, after complete dissolution of ds cDNA by incubation at 37° C for 30 minutes, 0.5 units of SI nuclease (product of PL Biochemical) was added.
(b) After 30 minutes of reaction at 37° C, 2 units of SI nuclease was further added and the reaction was allowed to proceed for further 15 minutes.
(c) 20 µl of 0.2 M EDTA was added to terminate the reaction, followed by addition of 120 µl of phenol-chloroform for deproteinization.
(d) Teh phenol layer was reextracted with TE buffer. Both the aqueous layers were combined and subjected to precipitation with ethanol to give ds cDNA.

(6) Sucrose density gradient centrifugation of ds cDNA

The ds cDNA obtained was then subjected to sucrose density gradient centrifugation (38,000 rpm, 4° C) and large molecular weight fractions were collected. The ultracentrifugation sedimentation pattern of urokinase ds cDNA is shown in Fig. 2 (indicated by ● - ● in the figure), together with the sedimentation pattern of globin ds cDNA as a marker (indicated by ▲ - ▲ in the figure). Urokinase ds cDNA shows a slight shift of the peak fraction to the bottom as compared with globin ds cDNA. Urokinase ds cDNA was also more abundant in large ds cDNA in fractions 11 to 15 and the neighboring fractions. Therefore, fractions 6-15 were collected and used as large ds cDNA. This large ds cDNA weighed 722 ng.

(7) Addition of poly(C) tail to ds cDNA

The C-tail addition reaction was conducted on the assumption that urokinase ds cDNA is on an average 1,500 bp in size. The C-tail reaction should ideally be conducted such that a poly(C) tail comprising 15 to 20 dCs is added to the 3' OH terminal of ds cDNA. The inventors used 5 units of terminal transferase and

carried out the reaction at 37° C. As a result, about 20 dCs were added as the C-tail.

In the same manner, a poly(G) tail comprising about 15 dGs was added to the PstI site of Escherichia coli plasmid pBR322 prepared separately. Equimolar amounts of both products were mixed and annealed under high ion strength conditions by gentle cooling from 70° C to 37° C.

(8) Transformation

The Escherichia coli RRI was transformed with the annealed DNA, giving about 70,000 tetracycline-resistant strains. The transformants obtained were examined for ampicillin susceptibility by the replica method. More than 90% of the transformants, i.e. about 60,000 colonies, exhibited $Ap^s$ (Ampicillin susceptibility) and $Tc^r$(Tetracycline-resistance), suggesting the insertion of cDNA.

(9) Cloning of urokinase gene

For cloning urokinase cDNA, an oligodeoxyribonucleotide mixture was synthesized based on the known amino acid sequence of Urinary Urokinase and transformant screening was conducted using this as a hybridization probe.

(a) Synthetic oligodeoxyribonucleotide probe

The 14-base synthetic oligodeoxyribonucleotide mixture used as a probe (hereinafter referred to as probe) was custom-synthesized by Nippon Zeon Co., Ltd. (Tokyo).

The nucleotide sequence of the probe used and the corresponding urokinase nucleotide sequence are shown in Table 4. The probe was drawn from the 73rd to 77th amino acids of human urine urokinase B chain. As is evident in Table 4, the probe is a mixture of 8 oligonucleotides.

**Table 4 Oligonucleotide probe used for isolating human urokinase cDNA**

| | |
|---|---|
| Amino acid sequence | 73 74 75 76 77 |
| | $NH_2$-Glu-Met-Lys-Phe-Glu-COOH |
| Codon | 5'-$GA^{G}_{A}$-AUG-$AA^{G}_{A}$-$UU^{U}_{C}$-$GA^{G}_{A}$-3' |
| 14-mer mixture | 3'-$CT^{T}_{C}$-TAC-$TT^{T}_{C}$-$AA^{G}_{A}$-CT-5' |

Note: In the middle line is shown all possible sequences of mRNA. In the lower line is shown the base sequence of the probe DNA complementary to said mRNA. The probe DNA is lacking in the third base in the codon for the 77th amino acid (Glu).

(b) Preparation of probe labelled with $^{32}P$ at 5' end

i) $^{32}P$ labelling of probe at 5' end

The 5' end of the probe was labelled with [Y-$^{32}P$] ATP (Amersham, Specific activity 5,000 ci/mMol) and T4 polynucleotide kinase. As regards the labelling conditions, the mole ratio of $^{32}P$-ATP to 5'-OH end was 5:1 and the reaction system was as follows:

Reaction mixture:

| | | |
|---|---|---|
| | Probe DNA | 6-80 pmol 5'-OH end |
| *1 | 10 x kinase buffer | 2 µl |
| | T4 polynucleotide kinase | 1 µl |
| *2 | [γ-$^{32}$P]ATP | 5 x mol of 5'-OH end |
| | $H_2O$ | To make the total 20 µl |

*1 10 x kinase buffer:

0.7 M Tris-HCl (pH 7.6)

0.1 M $MgCl_2$

50 mM DTT

The reaction was carried out at 37° C for 60-120 minutes. After completion of the reaction, 1 µl of 0.5 M EDTA (pH 8.0), 35 µl of 1M sodium chloride (in 10 mM Tris-HCl buffer and 1 mM EDTA) and 10.5 µl of 1 mg/ml tRNA solution and the final volume was made 35 µl, and the mixture was heat-treated at 70° C for 3 minutes and then applied to a NACS-52 minicolumn.

ii) Purification of $^{32}$P-labelled probe

The $^{32}$P-labelled probe was purified by applying the reaction mixture prepared in i) on to a NACS-52 minicolumn (BRL).

The efficiency of labelling was almost constant irrespective of the kind of probe. Supposing that the recovery from the column was 80%, the specific activity of the probe DNA was estimated at about 1.0 x $10^9$ cpm/µg of DNA.

(c) Colony hybridization

Using the probe mentioned above, screening for urokinase cDNA was conducted by colony hybridization.

i) The reagents and buffers used were as follows: Deionized formamide:

Formamide (500 ml) and Mixed-bed-resin (30 ml) were mixed and the mixture was stirred at 4° C for 2 hours, suction-filtered and stored at -80° C.

50 x Denhardt's solution:

Stored at -20° C after bacterial filtration.

20 x SSPE:

3.6 M NaCl

0.2 M $NaH_2PO_4$ (adjusted to pH 7.4 with 10 N NaOH)

20 mM EDTA

20 x SSC

Sonicated salmon sperm DNA

Prehybridization washing solution (hereinafter, PHWS):

50 mM Tris-HCl (pH 8.0)

1 M NaCl

1 mM EDTA

0.1% SDS

Prehybridization solution:

50% deionized formamide

5 x Denhardt's solution

5 x SSPE

0.1% SDS

100 µg/ml Sonicated DNA

Washing solution I (hereinafter, WS I):

5 x SSC

1 x Denhardt's solution

0.1% SDS

100 µg/ml tRNA
Washing solution II (hereinafter, WS II):
5 x SSC
0.1% SDS
Hybridization solution (hereinafter, HS):
5 x SSC
1 x Denhardt's solution
0.1% SDS
100 µg/ml tRNA
$^{32}$P-labelled probe
ii) Hybridization

The hybridization process can be summarized as follows:

Dry filter
↓
Washing (moistening the filter with PHWS at 42°C for 1 to 2 hours)
↓
Prehybridization (42°C, 4 to 6 hours)
↓

↓
Washing twice with WS I
↓
Hybridization (37°C, 20 hours, in $^{32}$P-labelled probe-containing HS)
↓
Washing with WS II (room temperature, 3 minutes, three times; then, 37°C, 3 minutes, three times)

Air drying, drying at 80° C under reduced pressure, and autoradiography at -70° C

Using 3.0 x 10$^{8}$ cpm of the $^{32}$P-labelled probe, 10,000 colonies (100 filters) were screened. The total amount of HS was 150 ml and therefore the probe concentration was 2.0 x 10$^{6}$ cpm/ml.

The hybridization was conducted at 37° C for 21 hours with occasional shaking. Thereafter, the filter was washed with WS II three times at room temperature and then three times at 37° C. The dried filter was placed on a filter paper (Whatman 3MM), and autoradiography was performed at -70° C for 2 days, followed by development. As a result, 65 colonies seemingly indicative of relatively high rate of hybridization were selected and again fixed on a filter for rescreening.

The 65 colonies selected were arranged on an L-agar plate (with pBR322 as a negative control), and 2 filters of the same kind were prepared. Hybridization was conducted with the probe using the filters in pairs,

the probe DNA concentration being 5.8 x $10^5$ cpm/ml/oligonucleotide species.

After 21 hours of hybridization at 37° C, the filters were washed with WS II. One filter was dried at 37° C directly after washing, while the other was further washed three times at 42° C for 3 minutes, followed by exposure of an X-ray film thereto.

In this hybridization experiments, four colonies were found to be indicative of hybridization with the probe DNA. After washing at 42° C, these 4 colonies were still clearly recognized as positive colonies.

For the 4 colonies selected, plasmid DNAs (named pUK1, 2, 3 and 4, respectively) were prepared and examined for the size of cDNA. Among the 4 plasmids pUK1 had the largest cDNA of about 1,900 bp. pUK3 and pUK4 were smaller than pUK1 and were about 1,300 bp and about 1,150 bp in size, respectively. pUK2 was the smallest cDNA about 170 bp in size. Since, in the restriction enzyme maps, pUK1, 3 and 4 showed overlapping with one another, they were considered to have cloned different regions of one and the same mRNA.

(10) Preparation of restriction enzyme maps

The restriction enzyme maps were prepared for comparison for the four plasmids selected in the above.

The restriction enzymes used were

AccI, AluI, AvaI, AvaII, BamHI, BclI, BglI, BglII, BstEII, ClaI, EcoRI, Fnu4HI, HaeIII, HincII, HindIII, KpnI, NcoI, MluI, PstI, PvuII, PsuI, SacI, SalI, Sau96I, ScaI, SmaI, StuI, XbaI and XhoI. The buffers used with these restriction enzymes are shown in Table 5.

For AvaI, RsaI buffer was used; for SacI, KpnI buffer; for StuI, SalI and XhoI, BamHI buffer; for NcoI and ScaI, BglII buffer; and, for MluI, HindIII buffer.

Table 5

| Enzyme | Final concentration of constituent | | | | | | |
|---|---|---|---|---|---|---|---|
| | Tris mM | pH | NaCl mM | $MgCl_2$ mM | $(NH_4)_2$ $SO_4$ mM | KCl mM | Mercapto-ethanol |
| Acc I | 10 | 7.5 | 60 | 7 | — | — | 5 |
| Alu I | 6 | 7.6 | 50 | 6 | | | 5 |
| Ava II | 6 | 8.0 | 60 | 10 | | | 5 |
| BamH I | 10 | 8.0 | 100 | 7 | | | 5 |
| Bcl I | 6 | 7.4 | | 10 | | 75 | - |
| Bgl I | 10 | 7.4 | 66 | 10 | | | 2 |
| Bgl II | 10 | 7.5 | 100 | 10 | | | 5 |
| BstE II | 6 | 7.9 | 150 | 6 | | | - |
| Cla I | 6 | 7.9 | 50 | 6 | | | 5 |
| EcoR I | 100 | 7.5 | 50 | 7 | - | - | 5 |
| Fna4H I | 6 | 7.4 | 6 | 6 | | 5 | 5 |
| Hae III | 10 | 7.5 | 60 | 7 | | | 5 |
| Hinc II | 10 | 8.0 | 60 | 7 | | | 5 |
| Hind III | 10 | 7.5 | 60 | 7 | | | 5 |
| Kpn I | 6 | 7.5 | 6 | 6 | | | - |
| Pst I | 20 | 7.5 | - | 10 | 50 | - | - |
| Pvu II | 6 | 7.5 | 60 | 6 | | | - |
| Rsa I | 6 | 8.0 | 50 | 12 | | | 5 |
| Sau96 I | 6 | 7.4 | 60 | 15 | | | 5 |
| Sma I | 6 | 8.0 | | 6 | | 20 | 5 |
| Xba I | 6 | 7.9 | 50 | 6 | | | |

As already mentioned hereinabove, the size of cDNA inserted in pUk1 is 1,900 bp and the largest among pUK1 through 4. The size of pUK3 and that of pUK4 are 1,300 bp and 1,150 bp, respectively. The cDNA of pUK2 is a fairly small and 170 bp in size. Therefore, for the three plasmids except pUK2, the restriction enzyme map of each cDNA was prepared. Table 6 show the number of restriction sites for each enzyme was determined (Table 6) and thus the distance between restriction enzyme sites was determined

for each enzyme. Upon cleavage with EcoRI, pUK1 and pUK4 gave fragments quite equal in size to each other (420 bp). Upon double digestion with BamHI and EcoRI and with BamHI and PstI, a fragment from pUK1 was entirely the same in size with one from pUK3. Therefore, taking the EcoRI site as a basis for pUK1 and pUK4 and the BamHI site as a standard for pUK1 and pUK3, other restriction enzyme sites of these plasmids were examined, whereby the restriction enzyme sites on cDNA of pUK1 were found to be almost in agreement in position with those on cDNA of pUK3 and of pUK4. On the supposition that the cDNA of pUK1, that of pUK3 and that of pUK4 are identical to one another, it was estimated that, in pUK1 and pUK4, the cDNA was inserted in pBR322 in the same direction and that, in pUK3, the cDNA was inserted in the opposite direction as compared with pUK1 and pUK4.

Since the cDNAs inserted in pUK1, pUK3 and pUK4 could be regarded as one and the same in view of the restriction enzyme sites occurring therein, the possibility that these three cDNAs each might be the urokinase cDNA became very great. The grounds are that, in spite of very low probability of plasmids having a sequence identical with the base sequence of the synthetic 14-mer DNA used in screening, 3 out of the 4 colonies found to be positive in the screening had the same cDNA, rendering high the probability that the cDNA owned by these positive colonies might have the desired sequence for urokinase; and that the position of the DNA base sequence synthesized as a probe on urokinase mRNA (Fig. 3) agreed with the place of overlapping among pUK1, pUK3 and pUK4 (Fig. 1). The DNA base sequence of part of pUK1 and of pUK4 was determined. The corresponding amino acid sequence deduced was in agreement with the amino acid sequence of urokinase.

Table 6

| Restriction enzyme | Number of restriction enzyme cleavage sites | | |
|---|---|---|---|
| | pUK1 | pUK3 | pUK4 |
| AccI | 2 | 2 | 1 |
| AvaI | 0 | 0 | 0 |
| BamHI | 1 | 1 | 0 |
| BclI | 1 | 1 | 1 |
| BglI | 0 | 0 | 1 |
| BglII | 1 | 0 | 1 |
| BstEII | 0 | 0 | 0 |
| ClaI | 0 | 0 | 0 |
| EcoRI | 2 | 1 | 2 |
| HincII | 0 | 0 | 0 |
| HindIII | 0 | 0 | 0 |
| NcoI | 2 | 1 | 1 |
| KpnI | 0 | 0 | 0 |
| MluI | 0 | 0 | 0 |
| PstI | 2 | 3 | 1 |
| PvuI | 0 | 0 | 0 |
| PvuII | 4 | 3 | 2 |
| RsaI | 1 | 0 | 2 |
| SacI | 0 | 0 | 0 |
| SalI | 0 | 0 | 0 |
| ScaI | 0 | 0 | 1 |
| SmaI | 0 | 0 | 0 |
| StuI | 1 | 1 | 0 |
| XbaI | - | - | 0 |
| XhoI | 0 | - | 0 |

(11) Determination of nucleotide sequence of urokinase cDNA

For the purpose of directly clarifying whether the plasmids obtained by the procedure mentioned in the preceding paragraph did contain the urokinase cDNA, the DNA base sequence was determined by the

Maxam-Gilbert method. Comparison of the possible amino acid sequence deducible from the DNA base sequence obtained with the known amino acid sequence of human urinary urokinase revealed that cloned cDNAs is a sequence for signal peptide-containing pro-UK.

(a) The materials and method used are as follows:

i) Plasmid DNA

The above-mentioned pUK1 and pUK4 were used.

ii) Restriction enzymes

AccI, BamHI, BglI, EcoRI, HpaII, PstI, PvuII, Sau3AI, ScaI and RsaI were from Takara Shuzo and BclI, NcoI and TaqI from NEB.

iii) Restriction enzyme map of plasmid DNA

The restriction enzyme map of each plasmid with the cDNA inserted therein was prepared by the procedure mentioned under the preceding paragraph (10).

iv) Preparation of DNA fragment for use in base sequence determination

The DNA base sequence of each cDNA was determined by the Maxam-Gilbert sequencing method [Proc. Natl. Acad Sci. U.S.A., 74, 560 (1977); Methods in Enzymol., 65, 499 (1980)].

Thus, the plasmid DNA was digested with an appropriate restriction enzyme and the 5'-end labelling was performed using [$\gamma$-$^{32}$P]ATP and T4-polynucleotide Kinase (Fig. 5). The DNA fragments recovered were determined for its nucleotide sequence.

v) Sequencing of DNA nucleotide sequence

The [$^{32}$P]-labelled DNA fragment was subjected to Maxam-Gilbert's limited chemical degradation [Proc. Natl. Acad. Sci. U.S.A., 74, 560 (1977); Methods in Enzymol., 65, 499 (1980)], followed by 8% and 20% 7M urea-polyacrylamide gel electrophoresis in the conventional manner and autoradiography at -70 °C for 2-3 days.

vi) Amino acid sequence of urokinase

Judgment as to whether the cDNA obtained was truly the urokinase cDNA was made by comparing the amino acid sequence deduced from the base sequence found with the known amino acid sequence of human urine urokinase [Hopper-Seyler's Z. physiol. Chem., 363, 1043 (1982)]. The following sequence (III) shows the total amino acid sequence each of human urokinase A-chain and B-chain.

Sequence (Ⅲ): the entire amino acid sequence each of human urokinase chain A and chain B

A-Chain

1 10 20
Ser-Asn-Glu-Leu-His-Glu-Val-Pro-Ser-Asn-Cys-Asp-Cys-Leu-Asn-Gly-Gly-Thr-Cys-Val-

21 30 40
Ser-Asn-Lys-Tyr-Phe-Ser-Asn-Ile-His-Trp-Cys-Asn-Cys-Pro-Lys-Lys-Phe-Gly-Gly-Gln-

41 50 60
His-Cys-Glu-Ile-Asp-Lys-Ser-Lys-Thr-Cys-Tyr-Glu-Gly-Asn-Gly-His-Phe-Tyr-Arg-Gly-

61 70 80
Lys-Ala-Ser-Thr-Asp-Thr-Met-Gly-Arg-Pro-Cys-Leu-Pro-Trp-Asn-Ser-Ala-Thr-Val-Leu-

81 90 100
Gln-Gln-Thr-Tyr-His-Ala-His-Arg-Ser-Asp-Ala-Leu-Gln-Leu-Gly-Leu-Gly-Lys-His-Asn-

101 110 120
Tyr-Cys-Arg-Asn-Pro-Asp-Asn-Arg-Arg-Arg-Pro-Trp-Cys-Tyr-Val-Gln-Val-Gly-Leu-Lys-

121 130 140
Pro-Leu-Val-Gln-Glu-Cys-Met-Val-His-Asp-Cys-Ala-Asp-Gly-Lys-Lys-Pro-Ser-Ser-Pro-

141 150 160
Pro-Glu-Glu-Leu-Lys-Phe-Gln-Cys-Gly-Gln-Lys-Thr-Leu-Arg-Pro-Arg-Phe- * * * * * *

B-Chain

1 10 20
Ile-Ile-Gly-Gly-Glu-Phe-Thr-Thr-Ile-Glu-Asn-Gln-Pro-Trp-Phe-Ala-Ala-Ile-Tyr-Arg-

21 30 40
Arg-His-Arg-Gly-Gly-Ser-Val-Thr-Tyr-Val-Cys-Gly-Gly-Ser-Leu-Ile-Ser-Pro-Cys-Trp-

(continued on the following page)

```
41                                      50                                      60
Val-Ile-Ser-Ala-Thr-His-Cys-Phe-Ile-Asp-Tyr-Pro-Lys-Lys-Glu-Asp-Tyr-Ile-Val-Tyr-

61                                      70                                      80
Leu-Gly-Arg-Ser-Arg-Leu-Asn-Ser-Asn-Thr-Gln-Gly-Glu-Met-Lys-Phe-Glu-Val-Glu-Asn-

81                                      90                                     100
Leu-Ile-Leu-His-Lys-Asp-Tyr-Ser-Ala-Asp-Thr-Leu-Ala-His-His-Asn-Asp-Ile-Ala-Leu-

101                                    110                                     120
Leu-Lys-Ile-Arg-Ser-Lys-Glu-Gly-Arg-Cys-Ala-Gln-Pro-Ser-Arg-Thr-Ile-Gln-Thr-Ile-

121                                    130                                     140
Cys-Leu-Pro-Ser-Met-Tyr-Asn-Asp-Pro-Gln-Phe-Gly-Thr-Ser-Cys-Glu-Ile-Thr-Gly-Phe-

141                                    150                                     160
Gly-Lys-Glu-Asn-Ser-Thr-Asp-Tyr-Leu-Tyr-Pro-Glu-Gln-Leu-Lys-Met-Thr-Val-Val-Lys-

161                                    170                                     180
Leu-Ile-Ser-His-Arg-Glu-Cys-Gln-Gln-Pro-His-Tyr-Tyr-Gly-Ser-Glu-Val-Thr-Thr-Lys-

181                                    190                                     200
Met-Leu-Cys-Ala-Ala-Asp-Pro-Gln-Trp-Lys-Thr-Asp-Ser-Cys-Gln-Gly-Asp-Ser-Gly-Gly-

201                                    210                                     220
Pro-Leu-Val-Cys-Ser-Leu-Gln-Gly-Arg-Met-Thr-Leu-Thr-Gly-Ile-Val-Ser-Trp-Gly-Arg-

221                                    230                                     240
Gly-Cys-Ala-Leu-Lys-Asp-Lys-Pro-Gly-Val-Tyr-Thr-Arg-Val-Ser-His-Phe-Leu-Pro-Trp-

241                                    250
Ile-Arg-Ser-His-Thr-Lys-Glu-Glu-Asn-Gly-Leu-Ala-Leu
```

vii) Restriction enzyme map of cDNA and sequencing
strategy for determining DNA base sequence

pUK1 and pUK4 have restriction enzyme maps overlapping each other. On the other hand, pUK18, which was selected by colony hybridization using the Nick-translated PstI fragment of pUK1, has a

restriction enzyme map overlapping with the map of pUK1 (Fig. 4). Therefore, the cDNAs of these plasmids are considered to have cloned different regions of the same mRNA and accordingly can be combinedly regarded as a single cDNA having a total length of about 2,250 base pairs, as shown in Fig. 5. According to this way of thinking, the sequencing strategy illustrated in Fig. 5 was established and the base sequence was determined.

[Results]

Comparison of the DNA base sequence obtained by sequencing with the amino acid sequence of human urinary urokinase [Sequence(III)] revealed that pUK1, 4 and 18 each contained part of pro-UK cDNA.

The following Sequence(IV) shows the total base sequence, from the 5'-end to the 3'-noncoding region, of the cDNA coding for an amino acid sequence equivalent to that of urokinase, as determided on the basis of the results of sequencing of individual fragments. Hereinafter, mention is made of the structure of the cDNA coding for an amino acid sequence equivalent to single-chain pro-urokinase as obtained in accordance with the present invention.

Sequence (IV): the base sequence of cDNA

```
5'  TCCACCTGTCCCCGCAGCGCCGGCTCGCGCCCTCCTGCCGCAGCCACCGAGCCGCCGTCTAGCGCCCCGACCTCGCCACC

    -20                                     -10                                     -1
    Met Arg Ala Leu Leu Ala Arg Leu Leu Leu Cys Val Leu Val Val Ser Asp Ser Lys Gly
    ATG AGA GCC CTG CTG GCG CGC CTG CTT CTC TGC GTC CTG GTC GTG AGC GAC TCC AAA GGC

    +1       10        20        30        40        50        60        70        80
    AGCAATGAACTTCATCAAGTTCCATCGAACTGTGACTGTCTAAATGGAGGAACATGTGTGTCCAACAAGTACTTCTCCAA
    Ser      .      Mature UROKINASE       .         .         .         .         .

    81       90        100       110       120       130       140       150       160
    CATTCACTGGTGCAACTGCCCAAAGAAATTCGGAGGGCAGCACTGTGAAATAGATAAGTCAAAAACCTGCTATGAGGGGA
    .        .         .         .         .         .         .         .         .

    161      170       180       190       200       210       220       230       240
    ATGGTCACTTTTACCGAGGAAAGGCCAGCACTGACACCATGGGCCGGCCCTGCCTGCCCTGGAACTCTGCCACTGTCCTT
    .        .         .         .         .         .         .         .         .

    241      250       260       270       280       290       300       310       320
    CAGCAAACGTACCATGCCCACAGATCTGATGCTCTTCAGCTGGGCCTGGGGAAACATAATTACTGCAGGAACCCAGACAA
    .        .         .         .         .         .         .         .         .

    321      330       340       350       360       370       380       390       400
    CCGGAGGCGACCCTGGTGCTATGTGCAGGTGGGCCTAAAGCCGCTTGTCCAAGAGTGCATGGTGCATGACTGCGCAGATG
    .        .         .         .         .         .         .         .         .

    401      410       420       430       440       450       460       470       480
    GAAAAAAGCCCTCCTCTCCTCCAGAAGAATTAAAATTTCAGTGTGGCCAAAAGACTCTGAGGCCCCGCTTTAAGATTATT
    .        .         .         .         .         .         .         .         .

    481      490       500       510       520       530       540       550       560
    GGGGGAGAATTCACCACCATCGAGAACCAGCCCTGGTTTGCGGCCATCTACAGGAGGCACCGGGGGGGCTCTGTCACCTA
    .        .         .         .         .         .         .         .         .
```

(continued on the following page)

```
561       570       580       590       600       610       620       630       640
CGTGTGTGGAGGCAGCCTCATCAGCCCTTGCTGGGTGATCAGCGCCACACACTGCTTCATTGATTACCCAAAGAAGGAGG

641       650       660       670       680       690       700       710       720
ACTACATCGTCTACCTGGGTCACTCAAGGCTTAACTCCAACACGCAAGGGGAGATGAAGTTTGAGGTGGAAAACCTCATC

721       730       740       750       760       770       780       790       800
CTACACAAGGACTACAGCGCTGACACGCTTGCTCACCACAACGACATTGCCTTGCTGAAGATCCGTTCCAAGGAGGGCAG

801       810       820       830       840       850       860       870       880
GTGTGCGCAGCCATCCCGGACTATACAGACCATCTGCCTGCCCTCGATGTATAACGATCCCCAGTTTGGCACAAGCTGTG

881       890       900       910       920       930       940       950       960
AGATCACTGGCTTTGGAAAAGAGAATTCTACCGACTATCTCTATCCGGAGCAGCTGAAGATGACTGTTGTGAAGCTGATT

961       970       980       990      1000      1010      1020      1030      1040
TCCCACCGGGAGTGTCAGCAGCCCCACTACTACGGCTCTGAAGTCACCACCAAAATGCTGTGTGCTGCTGACCCACAGTG

1041     1050      1060      1070      1080      1090      1100      1110      1120
GAAAACAGATTCCTGCCAGGGAGACTCAGGGGGACCCCTCGTCTGTTCCCTCCAAGGCCGCATGACTTTGACTGGAATTG

1121     1130      1140      1150      1160      1170      1180      1190      1200
TGAGCTGGGGCCGTGGATGTGCCCTGAAGGACAAGCCAGGCGTCTACACGAGAGTCTCACACTTCTTACCCTGGATCCGC
```

(continued on the following page)

```
1201      1210      1220      1230      1240      1250      1260      1270      1280
 AGTCACACCAAGGAAGAGAATGGCCTGGCCCTCTGAGGGTCCCCAGGGAGGAAACGGGCACCACCCGCTTTCTTGCTGGT
 .        .         .         .   Stop  .         .         .         .         .

1281      1290      1300      1310      1320      1330
 TGTCATTTTTGCAGTAGAGTCATCTCCATCAGCTGTAAGAAGAGACTGGGAAGAT ..... 3'
 .        .         .         .         .         .         .
```

i) 5'-Noncoding region and signal sequence

As oan be seen in Sequence (II), there is a 5'-noncoding region covering at least 79 bases upstream from the protein synthesis start codon ATG (Met) and the first 20 amino acids beginning with Met (ATG) are not found in the amino acid sequence of human urine urokinase [Sequence (III)]. Therefore, it was found that said 20 amino acids constitute a signal peptide required for secretion of the urokinase

molecule from the cell. The amino acid sequence beginning with the 21st amino acid, Ser, agreed with that of human urine urokinase A-chain.

In view of the above, it is evident that the cDNA obtained codes for pre-pro UK having a signal peptide comprising 20 amino acids.

ii) Cleavage site in pro-UK

Human urine urokinase, which is composed of two chains, A-chain and B-chain, is presumably formed by activation cleavage into two chains, under the action of protease, such as plasmin for instance, of a pro-urokinase molecule synthesized in and secreted from the cell. This was confirmed by sequencing of the cDNA of pUK1.

In sequence(II), it is seen that the carboxyl terminal amino acid sequence [the 157th amino acid the in Sequence (II)] of human urine urokinase A-chain is followed by Lys and further by the amino terminal amino acid sequence of B-chain, namely Ile-Ile-Gly-Gly-.....

This indicates that human urine urokinase is at first synthesized as a single-chain pro-urokinase composed of A-chain and B-chain coupled with other via Lys and that said pro-urokinase is cleaved at the Arg-Phe-Lys-Ile-Ile site with loss of Lys.

iii) Carboxyl terminal and 3'-noncoding region of cDNA

Sequence(II)shows the terminal portion of the coding region of the cDNA provided by the present invention and part of the 3'-noncoding region thereof. As shown in the Sequence(II), the amino acid sequence agreeing with the carboxyl terminal of human urine urokinase B-chain, such as Gly-Leu-Ala-Leu, is read out from this base sequence and, downstream therefrom, there occurs the protein synthesis termination codon TGA. Therefore, it was considered that the carboxyl terminal of human urine urokinase is identical with that of pro-UK according to the invention and that there is no polypeptide to be lost by processing.

On the other hand, the restriction enzyme map suggests that the 3'-noncoding region should be huge. Although only part of the 3'-noncoding region has so far been sequenced, the 3'-noncoding region hitherto obtained in the study of pUK18 is about 850 bp in size. However, pUK18 does not contain the poly(A) sequence characteristic of the 3'-terminal of eukaryotic mRNA, so that the 3'-noncoding region is possibly much longer.

(12) Construction of plasmid containing urokinase-coding region

pUK1 and pUK4 were digested with the restriction enzymes HindIII and BglII at 37°C for 1-2 hours according to the table in paragraph (10). A fragment of about 5.3 kb was isolated from pUK1, and a fragment of about 1.2 kb from pUK4. Both the fragments were ligated with each other by reaction in ligation buffer (20 mM Tris-HCl pH 7.5, 10 mM $MgCl_2$, 10 mM dithiothreitol, 0.5 mM ATP) containing 10 units of T4 DNA ligase at 20°C for 2 hours. The above steps are shown in Fig. 6. (In Fig. 6, E stands for EcoRI, H for HindIII, and B for BamHI). Resulted plasmid, named pUK33, containing about 2.2 kbp of pre-urokinase cDNA.

(13) Production of urokinase in animal cells

(a) Construction of vector for expression in animal cells

pSV-GI (Fig. 8) was constructed using, as the starting plasmids, pcDVI and pLI (Fig. 7) created by Okayama and Berg [Molec. and Cell. Biol., 3, 280-289 (1983)]. The pcDVI and pLI are hybrid plasmids of pBR322 DNA and SV40 DNA. They were obtained from PL Biochemicals (Pharmacia). For facilitating the insertion of urokinase-encoding DNA into these plasmids, pSV-GI was constructed by inserting a HindIII-PstI fragment of pLI into pcDVI at the HindIII-KpnI site thereof.

Thus, 4 µg of pLI was digested with PstI, the protruding 3'-end was rendered blunt by treatment with T4 DNA polymerase. Namely, 76 µl of reaction mixture No. 1 (4 µg of pLI, 8 µl of 10 x T4 polymerase buffer, 4.0 µl each of 2 mM dNTP solution, d $H_2O$) was heated at 65°C for 3 minutes and then quenched, 4 µl (10 U) of T4 polymerase was added to make the total volume 80 µl, and the reaction was allowed to proceed at 37°C for 5 minutes. Thereafter, 8 µl of 0.25 M EDTA (pH 8.0) and 80 µl of d $H_2O$ were added, the resulting mixture was subjected once to treatment with phenol-chroloform, and the aqueous layer was recovered. DNA was recovered by precipitation with ethanol, dried under reduced prssure, and subjected to KpnI linker ligation. The ligation reaction was carried out at 16°C overnight in a reaction mixture No. 2 (4 µg of dried DNA, 2 µl (2 µg) of 5'-P KpnI linker, 2.0 µl of 10 x ligase buffer, d $H_2O$) adding 2 µl (5 U) of T4 ligase. Thereafter, digestion was carried out with KpnI and then with HindIII, followed by 5% polyacrylamide gel electrophoresis (PAGE), whereby a DNA fragment of about 600 bp

was recovered.

Separately, pcDVI was digested with HindIII and KpnI, and a DNA fragment of about 2.7 kbp was isolated on 1% agarose gel. A 100-ng portion of the fragment was ligated with the above-mentioned pLI-derived DNA fragment of about 600 bp. The ligation was carried out by adding 2 µl (5.6 U) of T4 ligase to 8 µl of reaction mixture No. 3 (150 ng of pLI fragment DNA, 100 ng of pcDVI fragment DNA, 1.0 µl of 10 x T4 ligase buffer, d $H_2O$) and allowing the reaction to proceed at 16° C overnight. A 1/2 portion of the reaction mixture was used for performing transformation of E. coli HB101.

Using 12 colonies out of the transformants obtained, plasmid DNAs were prepared by the mini-prep method and examined by digestion with various restriction enzymes. All the plasmid DNAs were found to contain the desired plasmid. Therefore, using one strain, plasmid DNAs were prepared in large amounts and named pSV-GI, the restriction enzyme map thereof was prepared (Fig. 8). The pSV-GI obtained could be judged to have SV-40 early enhancer/promoter element and late 5'-splciing junction arranged upstream and late poly(A) signal and terminater arranged downstream, with the KpnI site as the cloning site and thus have elements involved in the functional mRNA synthesis in animal cells without any deficiency.

(b) Insertion of urokinase DNA sequence into vector

pUK33 (containing urokinase cDNA 2.2 kbp in total length) obtained in (12) was partially digested with PstI, and a 1.7 kbp fragment was isolated and inserted into KpuI site of the pSV-GI. Thus, 500 µl of reaction mixture No. 4 [100 µg of pUK33, 50 µl of 10 x PstI buffer, 10 µl (60 U) of PstI, d $H_2O$] was maintained at 37° C. After 10, 15 and 20 minutes of reaction, the reaction mixture was sampled in about 160 µl portions. The sampled reaction mixtures were heat-treated at 65° C for 5 minutes, combined and subjected to 1% agarose gel clectrophoresis, whereby about 10 µg of the 1.7 kbp containing pre-UK cDNA with its 5'-and 3'-noncoding sequence fragment was recovered. About 5 µg of the recovered fragment of about 1.7 kbp was treated with T4 DNA polymerase to render the protruding 3'-end blunt, and subjected to ligation with KpnI linker as m (13-a). The ligation product was digested with KpnI and then further subjected to ligation with KpnI-digested pSV-GI. The product was used for transformation of E. coli HB101, whereby pSV-GI-preUK (Fig. 9) was obtained.

As seen in Fig. 10 pSV-GI-preUK contains 5'-noncoding region of pro-UK cDNA, signal sequence and 3'-noncoding region as inserted downstream from SV-40 early promoter, and when introduced into appropriate recipient cells, the DNA can induce production of human pro-UK.

(c) Preparation of dominant selective marker-containing plasmid

Using the above pSV-GI, pSV-GI-Neo$^r$, a plasmid to be used as a dominant selective marker in transforming cells to be cultured, was constructed in the following manner.

The plasmid pNEO with the Tn5-derived Neo$^r$ gene clones therein [Southern, P. J. and Berg, J.: Molec, and Applied Genet., 1, 327-341 (1982)] was subjected to double digestion with BamHI and HindIII, followed by recovery of DNA fragments by precipitation with ethanol. The DNAs recovered were rendered blunt-ended by treatment with E. coli DNA polymerase I large fragment (Klenow fragment) in the presence of all 4 dNTPs. A blunt-ended 1.5 kb DNA fragment was separated and then the KpnI linker was ligated using T4 DNA ligase. After KpnI linker ligation, the DNA fragment was completely digested with 60 U of KpnI, and the desired Neo$^r$ fragment of about 1.5 kb with said linker joined thereto was recovered.

A 150-ng portion of the DNA fragment thus recovered was ligated with 20 ng of pSV-GI cleaved with KpnI in the above manner, and the ligation product was used for transformation of E. coli HB101. In this manner, pSV-GI-Neo$^r$ was obtained (Fig. 9).

(d) Preparation of recipient cell

Chinese hamster ovary cell [CHO-KI, ATCC CCL61 (purchased from Flow Labo. Inc.)] was used as the recipient cells. Cell culture was conducted in Ham's F-12 (Gibco) containing 10% FCS (fetal calf serum) (Boehringer Mannheim). The cells were maintained by subculture in Falcon 3028 flasks. Subconfluent cells were collected 2 days after inoculation by trypsinization, suspended in the above-mentioned culture medium and inoculated into Falcon #3003 (100 mm) culture dishes at an inoculum size of about 5 x $10^5$ cells/10ml/100mm. After 24 hours of incubation in a $CO_2$ incubator (5% $CO_2$-95% air), the cells were subjected to transformation. The number of cells per dish on that occasion was about 7.0 x $10^5$.

(e) Transformation

To the dished prepared in (d), there was added 1 ml per dish of a DNA $CaPO_4$ CO- precipitate containing 2.0 µg (as DNA)/ml of the plasmid DNA [pSV-GI-preUK and pSV-GI-Neo (mixing ration 100:1)] and 20 µg/ml of sonicated salmon sperm DNA as a carrier DNA, as prepared by the method of Osada et al. [Protein, Nucleic Acid and Enzyme, 28 (14), 1569-1581 (1983)]. About 18 hours after exposure to

DNA, the medium was exchanged. The incubation was then continued further 48 hours or more, followed by selection using antibiotics G-418.

(f) Selection of transformant cells

Since the urokinase gene itself is not "selectable", the transformant selection in eukaryotic cells was performed by cultivating the cells in G-418-containing medium (400 µg/ml) according to the method of Southern and Berg. [J. Molec, and Applied Genet., 1, 327-341 (1982)], with the Tn5-derived $Neo^r$ gene capable of giving G-418 resistance as the dominant selection marker. The G-418 used was from Gibco (Geneticin, Gibco, lot No. 75K6043 and 74N8040).

While exchanging the medium every 4 days, G-418-resistant colonies were cloned and grown. On day 10, 83 such colonies were obtained. The colonies obtained were examined for production of urokinase with RPHA reagent for human urokinase (Green Cross Corp.) and by fibrin plate assay. Primary screening with RPHA reagent revealed that about 25% of the G-418-resistant colonies produced human urokinase-like substance.

Fifteen strains showing RPHA-aggregation were tested for plasminogen activation activity by fibrin plate assay, whereby the activity was observed in the culture supernatant. This activity was neutralized by antihuman urokinase antibody purified by urokinase antigen column chromatography. The strain CHO-KI-G-68 seemingly highly capable of producing urokinase-like substance as estimated in the screening was chosen and re-cloned by low density culture, and two strains C-68-53 and C-68-61 were obtained.

Using the culture supernatnat of these strains, the human urokinase-like substance synthesized and secreted was examined for its properties. The clones C-68-53 and C-68-61 secreted human urokinase-like protein into the medium in a stable and constitutive manner for more than 100 days.

(g) Properties of human urokinase-like protein synthesized by CHO-KI cells

For said two clones, the human urokinase-like protein secreted into the medium was examined for its properties.

First, the production of human urokinase-like protein by each of these two clones was determined. Thus, each clone was inoculated into 25 $cm^2$ culture flasks (Falcon #3013) at an inoculum size of $5 \times 10^5$ cells/10 ml/flask. When the cells became confluent after 3 days of incubation, the medium was replaced with 5 ml/flask of maintenance medium containing 1% of FCS. After 24 hours of incubation in maintenance medium, the plasminogen activator activity in the culture supernatant was measured. The activity measurement by the fibrin plate method using human urinary high molecular weight urokinase as a reference indicated a yield of about 200 IU/ml/day for both the clones. Such activity was specifically neutralized by antihuman urokinase monoclonal antibody.

Next, the molecular weight of human urokinase-like protein produced by said clones was determined. Thus, the culture supernatant after 24 hours of incubation in maintenance medium was subjected to 12.5% SDS-PAGE. The band was blotted onto a nitrocellulose filter electrophoretically in 25 mM Tris-192 mM glycine (pH 8.3)/20% methanol. The filter was then blocked in 20 mM Tris-HCl (pH 7.5)/500mM NaCl containing 3% of gelatin at room temperature for 60 minutes and subjected to treatment with rabbit antihuman urokinase IgG purified by means of a human urokinase antigen column and protein A Sepharose as a primary antibody. Thus the human Urokinase band was specificially identified by using immuno-Blot™ assay system (Bio-Rad).

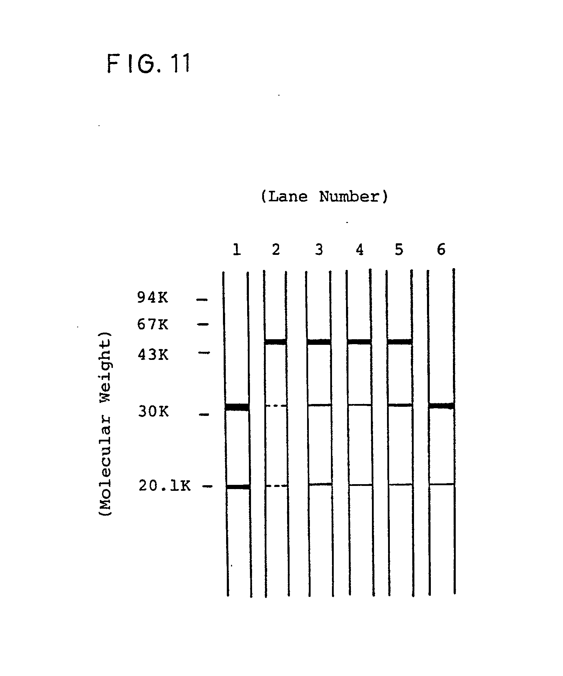

When the samples were used after reduction treatment with 2-mercaptoethanol, the culture supernatants of both the clones gave three bands, a main band 54 K in molecular weight and subbands 34 K and 20 K in molecular weight. Simultaneous electrophoresis of human urine-derived two-chain high molecular UK (as a reference) and the culture supernatant of pro-UK-producing human kidney-derived cell live revealed that the 54 K, 34 K and 20 K bands from the culture supernatant from both C-68-53 and C-68-61 cells agreed in molecular weight and reactivity to anti-UK IgG with the human natural-type single-chain pro-UK (54 K) band and the 34 K and 20 K bands from double-chain high molecular UK, respectively, no distinction could be made. (Fig. 11)

The above results indicated that, in both the clones, glycosylated single-chain pro-UK having a molecular weight of 54 K is synthesized and secreted, like in the case of said natural type, and part of it is converted secondarily to double-chain UK in the culture medium.

Therefore, the presence or absence of the sugar chain in the human pro-UK synthesized and secreted by both the clones was then examined by column chromatography using ConA-Sepharose 4B (Pharmacia).

Thus, the clones were cultivated until cells became confluent. The cells were washed twice with Dulbecco's PBS (phosphate-buffered Saline) (+) (Nissui) and then grown in 5 ml of proline-added, Met-free Eagle's MEM medium (product of Nissui) containing 1% of FCS dialyzed against PBS(+), together with 200 µCi/ml of [$^{35}$S)-methionine, for 20 hours.

The [$^{35}S$]-labelled culture fluid was applied to an anti-human urokinase monoclonal antibody column (5 ml). The column was washed well with 0.04 M sodium phosphate buffer (pH 8.0)/ 0.03 M NaCl and then eluted with glycine-HCl (pH 7.5), whereby $^{35}S$-labelled recombinant human urokinase was recovered. The eluate $^{35}S$-labelled human urokinase was neutralized with 1 M Tris, dialyzed against 20 mM sodium phosphate buffer pH 7.4/150 mM NaCl and applied to a ConA-Sepharose 4B (Pharmacia) column. After washing with buffer, elution was performed with sodium phosphate buffer (pH 7.4) containing 0.1 M methyl-α-D-mannoside. Most of the radioactivity eluted from the anti-UK monoclonal antibody column was adsorbed on ConA-Sepharose and specifically eluted with mannoside.

The above results indicate that the recombinant urokinase synthesized and secreted by both the clones is a glycoprotein purifiable by means of an anti-human urokinase monoclonal antibody column.

The above results also indicate that the human pro-UK cDNA introduced into CHO-KI can be transcribed in a normal manner and the pro-UK is synthesized and secreted as a glycoprotein having a molecular weight equivalent to that of the natural-type pro-UK and having reactivity to monoclonal anti human Urokinase antibodies.

## Claims

1. A method of producing glycosylated single-chain pro-urokinase which comprises cultivating animal cells transformed with a plasmid with the DNA inserted therein, said DNA having the sequence

Sequence(I): the base sequence of the structural gene for pro-UK

```
 1                                          10                                         20
Ser-Asn-Glu-Leu-His-Gln-Val-Pro-Ser-Asn-Cys-Asp-Cys-Leu-Asn-Gly-Gly-Thr-Cys-Val-
AGC AAT GAA CTT CAT CAA GTT CCA TCG AAC TGT GAC TGT CTA AAT GGA GGA ACA TGT GTG

 21                                         30                                         40
Ser-Asn-Lys-Tyr-Phe-Ser-Asn-Ile-His-Trp-Cys-Asn-Cys-Pro-Lys-Lys-Phe-Gly-Gly-Gln-
TCC AAC AAG TAC TTC TCC AAC ATT CAC TGG TGC AAC TGC CCA AAG AAA TTC GGA GGG CAG

 41                                         50                                         60
His-Cys-Glu-Ile-Asp-Lys-Ser-Lys-Thr-Cys-Tyr-Glu-Gly-Asn-Gly-His-Phe-Tyr-Arg-Gly-
CAC TGT GAA ATA GAT AAG TCA AAA ACC TGC TAT GAG GGG AAT GGT CAC TTT TAC CGA GGA

 61                                         70                                         80
Lys-Ala-Ser-Thr-Asp-Thr-Met-Gly-Arg-Pro-Cys-Leu-Pro-Trp-Asn-Ser-Ala-Thr-Val-Leu-
AAG GCC AGC ACT GAC ACC ATG GGC CGG CCC TGC CTG CCC TGG AAC TCT GCC ACT GTC CTT

 81                                         90                                        100
Gln-Gln-Thr-Tyr-His-Ala-His-Arg-Ser-Asp-Ala-Leu-Gln-Leu-Gly-Leu-Gly-Lys-His-Asn-
CAG CAA ACG TAC CAT GCC CAC AGA TCT GAT GCT CTT CAG CTG GGC CTG GGG AAA CAT AAT

101                                        110                                        120
Tyr-Cys-Arg-Asn-Pro-Asp-Asn-Arg-Arg-Arg-Pro-Trp-Cys-Tyr-Val-Gln-Val-Gly-Leu-Lys-
TAC TGC AGG AAC CCA GAC AAC CGG AGG CGA CCC TGG TGC TAT GTG CAG GTG GGC CTA AAG

121                                        130                                        140
Pro-Leu-Val-Gln-Glu-Cys-Met-Val-His-Asp-Cys-Ala-Asp-Gly-Lys-Lys-Pro-Ser-Ser-Pro-
CCG CTT GTC CAA GAG TGC ATG GTG CAT GAC TGC GCA GAT GGA AAA AAG CCC TCC TCT CCT

141                                        150                                        160
Pro-Glu-Glu-Leu-Lys-Phe-Gln-Cys-Gly-Gln-Lys-Thr-Leu-Arg-Pro-Arg-Phe-Lys-Ile-Ile-
CCA GAA GAA TTA AAA TTT CAG TGT GGC CAA AAG ACT CTG AGG CCC CGC TTT AAG ATT ATT
```

(continued on the following page)

```
161                                     170                                          180
Gly-Gly-Glu-Phe-Thr-Thr-Ile-Glu-Asn-Gln-Pro-Trp-Phe-Ala-Ala-Ile-Tyr-Arg-Arg-His-
GGG GGA GAA TTC ACC ACC ATC GAG AAC CAG CCC TGG TTT GCG GCC ATC TAC AGG AGG CAC

181                                     190                                          200
Arg-Gly-Gly-Ser-Val-Thr-Tyr-Val-Cys-Gly-Gly-Ser-Leu-Ile-Ser-Pro-Cys-Trp-Val-Ile-
CGG GGG GGC TCT GTC ACC TAC GTG TGT GGA GGC AGC CTC ATC AGC CCT TGC TGG GTG ATC

201                                     210                                          220
Ser-Ala-Thr-His-Cys-Phe-Ile-Asp-Tyr-Pro-Lys-Lys-Glu-Asp-Tyr-Ile-Val-Tyr-Leu-Gly-
AGC GCC ACA CAC TGC TTC ATT GAT TAC CCA AAG AAG GAG GAC TAC ATC GTC TAC CTG GGT

221                                     230                                          240
Arg-Ser-Arg-Leu-Asn-Ser-Asn-Thr-Gln-Gly-Glu-Met-Lys-Phe-Glu-Val-Glu-Asn-Leu-Ile-
CGC TCA AGG CTT AAC TCC AAC ACG CAA GGG GAG ATG AAG TTT GAG GTG GAA AAC CTC ATC

241                                     250                                          260
Leu-His-Lys-Asp-Tyr-Ser-Ala-Asp-Thr-Leu-Ala-His-His-Asn-Asp-Ile-Ala-Leu-Leu-Lys-
CTA CAC AAG GAC TAC AGC GCT GAC ACG CTT GCT CAC CAC AAC GAC ATT GCC TTG CTG AAG

261                                     270                                          280
Ile-Arg-Ser-Lys-Glu-Gly-Arg-Cys-Ala-Gln-Pro-Ser-Arg-Thr-Ile-Gln-Thr-Ile-Cys-Leu-
ATC CGT TCC AAG GAG GGC AGG TGT GCG CAG CCA TCC CGG ACT ATA CAG ACC ATC TGC CTG

281                                     290                                          300
Pro-Ser-Met-Tyr-Asn-Asp-Pro-Gln-Phe-Gly-Thr-Ser-Cys-Glu-Ile-Thr-Gly-Phe-Gly-Lys-
CCC TCG ATG TAT AAC GAT CCC CAG TTT GGC ACA AGC TGT GAG ATC ACT GGC TTT GGA AAA

301                                     310                                          320
Glu-Asn-Ser-Thr-Asp-Tyr-Leu-Tyr-Pro-Glu-Gln-Leu-Lys-Met-Thr-Val-Val-Lys-Leu-Ile-
GAG AAT TCT ACC GAC TAT CTC TAT CCG GAG CAG CTG AAG ATG ACT GTT GTG AAG CTG ATT

321                                     330                                          340
Ser-His-Arg-Glu-Cys-Gln-Gln-Pro-His-Tyr-Tyr-Gly-Ser-Glu-Val-Thr-Thr-Lys-Met-Leu-
TCC CAC CGG GAG TGT CAG CAG CCC CAC TAC TAC GGC TCT GAA GTC ACC ACC AAA ATG CTG
```

(continued on the following page)

```
341                                     350                                     360
Cys-Ala-Ala-Asp-Pro-Gln-Trp-Lys-Thr-Asp-Ser-Cys-Gln-Gly-Asp-Ser-Gly-Gly-Pro-Leu-
TGT GCT GCT GAC CCA CAG TGG AAA ACA GAT TCC TGC CAG GGA GAC TCA GGG GGA CCC CTC

361                                     370                                     380
Val-Cys-Ser-Leu-Gln-Gly-Arg-Met-Thr-Leu-Thr-Gly-Ile-Val-Ser-Trp-Gly-Arg-Gly-Cys-
GTC TGT TCC CTC CAA GGC CGC ATG ACT TTG ACT GGA ATT GTG AGC TGG GGC CGT GGA TGT

381                                     390                                     400
Ala-Leu-Lys-Asp-Lys-Pro-Gly-Val-Tyr-Thr-Arg-Val-Ser-His-Phe-Leu-Pro-Trp-Ile-Arg-
GCC CTG AAG GAC AAG CCA GGC GTC TAC ACG AGA GTC TCA CAC TTC TTA CCC TGG ATC CGC

401                                     410
Ser-His-Thr-Lys-Glu-Glu-Asn-Gly-Leu-Ala-Leu
AGT CAC ACC AAG GAA GAG AAT GGC CTG GCC CTC
```

and being prepared by using as the template mRNA obtained from cells of an established human kidney-derived cell line.

2. A method of producing glycosylated single-chain pro-urokinase as claimed in Claim 1, wherein the glycosylated single-chain pro-urokinase has a molecular weight of about 54,000.

3. A method of producing glycosylated single-chain pro-urokinase according to Claims 1 and 2, wherein

the animal cells are Chinese hamster ovary cells.

**Revendications**

1. Procédé de préparation de pro-uro-kinase glycosylée simple-brin, qui comprend la culture de cellules animales transformées en présence d'un plasmide contenant le DNA correspondant, ledit DNA ayant la séquence :

```
Sequence(I): La séquence de base du gène de structure pour pro-UK

1                                          10                                            20
Ser-Asn-Glu-Leu-His-Gln-Val-Pro-Ser-Asn-Cys-Asp-Cys-Leu-Asn-Gly-Gly-Thr-Cys-Val-
AGC AAT GAA CTT CAT CAA GTT CCA TCG AAC TGT GAC TGT CTA AAT GGA GGA ACA TGT GTG

21                                         30                                            40
Ser-Asn-Lys-Tyr-Phe-Ser-Asn-Ile-His-Trp-Cys-Asn-Cys-Pro-Lys-Lys-Phe-Gly-Gly-Gln-
TCC AAC AAG TAC TTC TCC AAC ATT CAC TGG TGC AAC TGC CCA AAG AAA TTC GGA GGG CAG

41                                         50                                            60
His-Cys-Glu-Ile-Asp-Lys-Ser-Lys-Thr-Cys-Tyr-Glu-Gly-Asn-Gly-His-Phe-Tyr-Arg-Gly-
CAC TGT GAA ATA GAT AAG TCA AAA ACC TGC TAT GAG GGG AAT GGT CAC TTT TAC CGA GGA

61                                         70                                            80
Lys-Ala-Ser-Thr-Asp-Thr-Met-Gly-Arg-Pro-Cys-Leu-Pro-Trp-Asn-Ser-Ala-Thr-Val-Leu-
AAG GCC AGC ACT GAC ACC ATG GGC CGG CCC TGC CTG CCC TGG AAC TCT GCC ACT GTC CTT

81                                         90                                           100
Gln-Gln-Thr-Tyr-His-Ala-His-Arg-Ser-Asp-Ala-Leu-Gln-Leu-Gly-Leu-Gly-Lys-His-Asn-
CAG CAA ACG TAC CAT GCC CAC AGA TCT GAT GCT CTT CAG CTG GGC CTG GGG AAA CAT AAT

101                                       110                                           120
Tyr-Cys-Arg-Asn-Pro-Asp-Asn-Arg-Arg-Arg-Pro-Trp-Cys-Tyr-Val-Gln-Val-Gly-Leu-Lys-
TAC TGC AGG AAC CCA GAC AAC CGG AGG CGA CCC TGG TGC TAT GTG CAG GTG GGC CTA AAG

121                                       130                                           140
Pro-Leu-Val-Gln-Glu-Cys-Met-Val-His-Asp-Cys-Ala-Asp-Gly-Lys-Lys-Pro-Ser-Ser-Pro-
CCG CTT GTC CAA GAG TGC ATG GTG CAT GAC TGC GCA GAT GGA AAA AAG CCC TCC TCT CCT

141                                       150                                           160
Pro-Glu-Glu-Leu-Lys-Phe-Gln-Cys-Gly-Gln-Lys-Thr-Leu-Arg-Pro-Arg-Phe-Lys-Ile-Ile-
CCA GAA GAA TTA AAA TTT CAG TGT GGC CAA AAG ACT CTG AGG CCC CGC TTT AAG ATT ATT
```

(suite sur la page suivante)

```
161                                     170                                     180
Gly-Gly-Glu-Phe-Thr-Thr-Ile-Glu-Asn-Gln-Pro-Trp-Phe-Ala-Ala-Ile-Tyr-Arg-Arg-His-
GGG GGA GAA TTC ACC ACC ATC GAG AAC CAG CCC TGG TTT GCG GCC ATC TAC AGG AGG CAC

181                                     190                                     200
Arg-Gly-Gly-Ser-Val-Thr-Tyr-Val-Cys-Gly-Gly-Ser-Leu-Ile-Ser-Pro-Cys-Trp-Val-Ile-
CGG GGG GGC TCT GTC ACC TAC GTG TGT GGA GGC AGC CTC ATC AGC CCT TGC TGG GTG ATC

201                                     210                                     220
Ser-Ala-Thr-His-Cys-Phe-Ile-Asp-Tyr-Pro-Lys-Lys-Glu-Asp-Tyr-Ile-Val-Tyr-Leu-Gly-
AGC GCC ACA CAC TGC TTC ATT GAT TAC CCA AAG AAG GAG GAC TAC ATC GTC TAC CTG GGT

221                                     230                                     240
Arg-Ser-Arg-Leu-Asn-Ser-Asn-Thr-Gln-Gly-Glu-Met-Lys-Phe-Glu-Val-Glu-Asn-Leu-Ile-
CGC TCA AGG CTT AAC TCC AAC ACG CAA GGG GAG ATG AAG TTT GAG GTG GAA AAC CTC ATC

241                                     250                                     260
Leu-His-Lys-Asp-Tyr-Ser-Ala-Asp-Thr-Leu-Ala-His-His-Asn-Asp-Ile-Ala-Leu-Leu-Lys-
CTA CAC AAG GAC TAC AGC GCT GAC ACG CTT GCT CAC CAC AAC GAC ATT GCC TTG CTG AAG

261                                     270                                     280
Ile-Arg-Ser-Lys-Glu-Gly-Arg-Cys-Ala-Gln-Pro-Ser-Arg-Thr-Ile-Gln-Thr-Ile-Cys-Leu-
ATC CGT TCC AAG GAG GGC AGG TGT GCG CAG CCA TCC CGG ACT ATA CAG ACC ATC TGC CTG

281                                     290                                     300
Pro-Ser-Met-Tyr-Asn-Asp-Pro-Gln-Phe-Gly-Thr-Ser-Cys-Glu-Ile-Thr-Gly-Phe-Gly-Lys-
CCC TCG ATG TAT AAC GAT CCC CAG TTT GGC ACA AGC TGT GAG ATC ACT GGC TTT GGA AAA

301                                     310                                     320
Glu-Asn-Ser-Thr-Asp-Tyr-Leu-Tyr-Pro-Glu-Gln-Leu-Lys-Met-Thr-Val-Val-Lys-Leu-Ile-
GAG AAT TCT ACC GAC TAT CTC TAT CCG GAG CAG CTG AAG ATG ACT GTT GTG AAG CTG ATT

321                                     330                                     340
Ser-His-Arg-Glu-Cys-Gln-Gln-Pro-His-Tyr-Tyr-Gly-Ser-Glu-Val-Thr-Thr-Lys-Met-Leu-
TCC CAC CGG GAG TGT CAG CAG CCC CAC TAC TAC GGC TCT GAA GTC ACC ACC AAA ATG CTG
```

(suite sur la page suivante)

```
341                                         350
Cys-Ala-Asp-Pro-Gln-Trp-Lys-Thr-Asp-Ser-Cys-Gln-Gly-Asp-Ser-Gly-Gly-Pro-Leu-
TGT GCT GAC CCA CAG TGG AAA ACA GAT TCC TGC CAG GGA GAC TCA GGG GGA CCC CTC
                                                                       360

361                                         370
Val-Cys-Ser-Leu-Gln-Gly-Arg-Met-Thr-Leu-Thr-Gly-Ile-Val-Ser-Trp-Gly-Arg-Gly-Cys-
GTC TGT TCC CTC CAA GGC CGC ATG ACT TTG ACT GGA ATT GTG AGC TGG GGC CGT GGA TGT
                                                                           380

381                                         390
Ala-Leu-Lys-Asp-Lys-Pro-Gly-Val-Tyr-Thr-Arg-Val-Ser-His-Phe-Leu-Pro-Trp-Ile-Arg-
GCC CTG AAG GAC AAG CCA GGC GTC TAC ACG AGA GTC TCA CAC TTC TTA CCC TGG ATC CGC
                                                                           400

401                                         410
Ser-His-Thr-Lys-Glu-Glu-Asn-Gly-Leu-Ala-Leu
AGT CAC ACC AAG GAA GAG AAT GGC CTG GCC CTC
```

et étant préparé par l'utilisation d'un brin mRNA matrice, obtenu à partir de cellules d'une lignée cellulaire établie dérivant du rein humain.

**2.** Procédé de préparation de pro-urokinase glycosylée simple brin selon la revendication 1, caractérisé en ce que la pro-uro-kinase glycosylée simple brin a un poids moléculaire d'environ 54 000.

**3.** Procédé de préparation de pro-uro-kinase glycosylée simple brin selon l'une des revendications 1 et 2, caractérisé en ce que les cellules animales sont des cellules d'ovaires d'hamsters chinois.

## Patentansprüche

1. Verfahren zur Herstellung glycosylierter einkettiger Pro-Urokinase, umfassend die Kultivierung von Tierzellen, die mit einem Plasmid mit der eingesetzten DNA transformiert wurden, wobei die DNA die Sequenz

Sequenz (I): Basensequenz des Strukturgens für Pro-UK

```
1                                       10                                      20
Ser-Asn-Glu-Leu-His-Gln-Val-Pro-Ser-Asn-Cys-Asp-Cys-Leu-Asn-Gly-Gly-Thr-Cys-Val-
AGC AAT GAA CTT CAT CAA GTT CCA TCG AAC TGT GAC TGT CTA AAT GGA GGA ACA TGT GTG

21                                      30                                      40
Ser-Asn-Lys-Tyr-Phe-Ser-Asn-Ile-His-Trp-Cys-Asn-Cys-Pro-Lys-Lys-Phe-Gly-Gly-Gln-
TCC AAC AAG TAC TTC TCC AAC ATT CAC TGG TGC AAC TGC CCA AAG AAA TTC GGA GGG CAG

41                                      50                                      60
His-Cys-Glu-Ile-Asp-Lys-Ser-Lys-Thr-Cys-Tyr-Glu-Gly-Asn-Gly-His-Phe-Tyr-Arg-Gly-
CAC TGT GAA ATA GAT AAG TCA AAA ACC TGC TAT GAG GGG AAT GGT CAC TTT TAC CGA GGA

61                                      70                                      80
Lys-Ala-Ser-Thr-Asp-Thr-Met-Gly-Arg-Pro-Cys-Leu-Pro-Trp-Asn-Ser-Ala-Thr-Val-Leu-
AAG GCC AGC ACT GAC ACC ATG GGC CGG CCC TGC CTG CCC TGG AAC TCT GCC ACT GTC CTT

81                                      90                                      100
Gln-Gln-Thr-Tyr-His-Ala-His-Arg-Ser-Asp-Ala-Leu-Gln-Leu-Gly-Leu-Gly-Lys-His-Asn-
CAG CAA ACG TAC CAT GCC CAC AGA TCT GAT GCT CTT CAG CTG GGC CTG GGG AAA CAT AAT

101                                     110                                     120
Tyr-Cys-Arg-Asn-Pro-Asp-Asn-Arg-Arg-Arg-Pro-Trp-Cys-Tyr-Val-Gln-Val-Gly-Leu-Lys-
TAC TGC AGG AAC CCA GAC AAC CGG AGG CGA CCC TGG TGC TAT GTG CAG GTG GGC CTA AAG

121                                     130                                     140
Pro-Leu-Val-Gln-Glu-Cys-Met-Val-His-Asp-Cys-Ala-Asp-Gly-Lys-Lys-Pro-Ser-Ser-Pro-
CCG CTT GTC CAA GAG TGC ATG GTG CAT GAC TGC GCA GAT GGA AAA AAG CCC TCC TCT CCT

141                                     150                                     160
Pro-Glu-Glu-Leu-Lys-Phe-Gln-Cys-Gly-Gln-Lys-Thr-Leu-Arg-Pro-Arg-Phe-Lys-Ile-Ile-
CCA GAA GAA TTA AAA TTT CAG TGT GGC CAA AAG ACT CTG AGG CCC CGC TTT AAG ATT ATT
```

(Fortsetzung nächste Seite)

```
161                                     170                                     180
Gly-Gly-Glu-Phe-Thr-Thr-Ile-Glu-Asn-Gln-Pro-Trp-Phe-Ala-Ala-Ile-Tyr-Arg-Arg-His-
GGG GGA GAA TTC ACC ACC ATC GAG AAC CAG CCC TGG TTT GCG GCC ATC TAC AGG AGG CAC

181                                     190                                     200
Arg-Gly-Gly-Ser-Val-Thr-Tyr-Val-Cys-Gly-Gly-Ser-Leu-Ile-Ser-Pro-Cys-Trp-Val-Ile-
CGG GGG GGC TCT GTC ACC TAC GTG TGT GGA GGC AGC CTC ATC AGC CCT TGC TGG GTG ATC

201                                     210                                     220
Ser-Ala-Thr-His-Cys-Phe-Ile-Asp-Tyr-Pro-Lys-Lys-Glu-Asp-Tyr-Ile-Val-Tyr-Leu-Gly-
AGC GCC ACA CAC TGC TTC ATT GAT TAC CCA AAG AAG GAG GAC TAC ATC GTC TAC CTG GGT

221                                     230                                     240
Arg-Ser-Arg-Leu-Asn-Ser-Asn-Thr-Gln-Gly-Glu-Met-Lys-Phe-Glu-Val-Glu-Asn-Leu-Ile-
CGC TCA AGG CTT AAC TCC AAC ACG CAA GGG GAG ATG AAG TTT GAG GTG GAA AAC CTC ATC

241                                     250                                     260
Leu-His-Lys-Asp-Tyr-Ser-Ala-Asp-Thr-Leu-Ala-His-His-Asn-Asp-Ile-Ala-Leu-Leu-Lys-
CTA CAC AAG GAC TAC AGC GCT GAC ACG CTT GCT CAC CAC AAC GAC ATT GCC TTG CTG AAG

261                                     270                                     280
Ile-Arg-Ser-Lys-Glu-Gly-Arg-Cys-Ala-Gln-Pro-Ser-Arg-Thr-Ile-Gln-Thr-Ile-Cys-Leu-
ATC CGT TCC AAG GAG GGC AGG TGT GCG CAG CCA TCC CGG ACT ATA CAG ACC ATC TGC CTG

281                                     290                                     300
Pro-Ser-Met-Tyr-Asn-Asp-Pro-Gln-Phe-Gly-Thr-Ser-Cys-Glu-Ile-Thr-Gly-Phe-Gly-Lys-
CCC TCG ATG TAT AAC GAT CCC CAG TTT GGC ACA AGC TGT GAG ATC ACT GGC TTT GGA AAA

301                                     310                                     320
Glu-Asn-Ser-Thr-Asp-Tyr-Leu-Tyr-Pro-Glu-Gln-Leu-Lys-Met-Thr-Val-Val-Lys-Leu-Ile-
GAG AAT TCT ACC GAC TAT CTC TAT CCG GAG CAG CTG AAG ATG ACT GTT GTG AAG CTG ATT

321                                     330                                     340
Ser-His-Arg-Glu-Cys-Gln-Gln-Pro-His-Tyr-Tyr-Gly-Ser-Glu-Val-Thr-Thr-Lys-Met-Leu-
TCC CAC CGG GAG TGT CAG CAG CCC CAC TAC TAC GGC TCT GAA GTC ACC ACC AAA ATG CTG
```

(Fortsetzung nächste Seite)

```
341                                     350                                     360
Cys-Ala-Ala-Asp-Pro-Gln-Trp-Lys-Thr-Asp-Ser-Cys-Gln-Gly-Asp-Ser-Gly-Gly-Pro-Leu-
TGT GCT GCT GAC CCA CAG TGG AAA ACA GAT TCC TGC CAG GGA GAC TCA GGG GGA CCC CTC

361                                     370                                     380
Val-Cys-Ser-Leu-Gln-Gly-Arg-Met-Thr-Leu-Thr-Gly-Ile-Val-Ser-Trp-Gly-Arg-Gly-Cys-
GTC TGT TCC CTC CAA GGC CGC ATG ACT TTG ACT GGA ATT GTG AGC TGG GGC CGT GGA TGT

381                                     390                                     400
Ala-Leu-Lys-Asp-Lys-Pro-Gly-Val-Tyr-Thr-Arg-Val-Ser-His-Phe-Leu-Pro-Trp-Ile-Arg-
GCC CTG AAG GAC AAG CCA GGC GTC TAC ACG AGA GTC TCA CAC TTC TTA CCC TGG ATC CGC

401                                     410
Ser-His-Thr-Lys-Glu-Glu-Asn-Gly-Leu-Ala-Leu
AGT CAC ACC AAG GAA GAG AAT GGC CTG GCC CTC
```

besitzt und hergestellt wird durch Verwendung einer mRNA als Matrize, die aus Zellen einer etablierten, aus Human-Nieren abgeleiteten Zellinie erhalten wurden.

2. Verfahren zur Herstellung glycosylierter einkettiger Pro-Urokinase nach Anspruch 1, wobei die glycosylierte einkettige Pro-Urokinase ein Molekulargewicht von etwa 54 000 besitzt.

3. Verfahren zur Herstellung glycosylierter einkettiger Pro-Urokinase nach den Ansprüchen 1 oder 2,

wobei es sich bei den Tierzellen um Ovar-Zellen von chinesischen Hamstern handelt.

FIG. 1

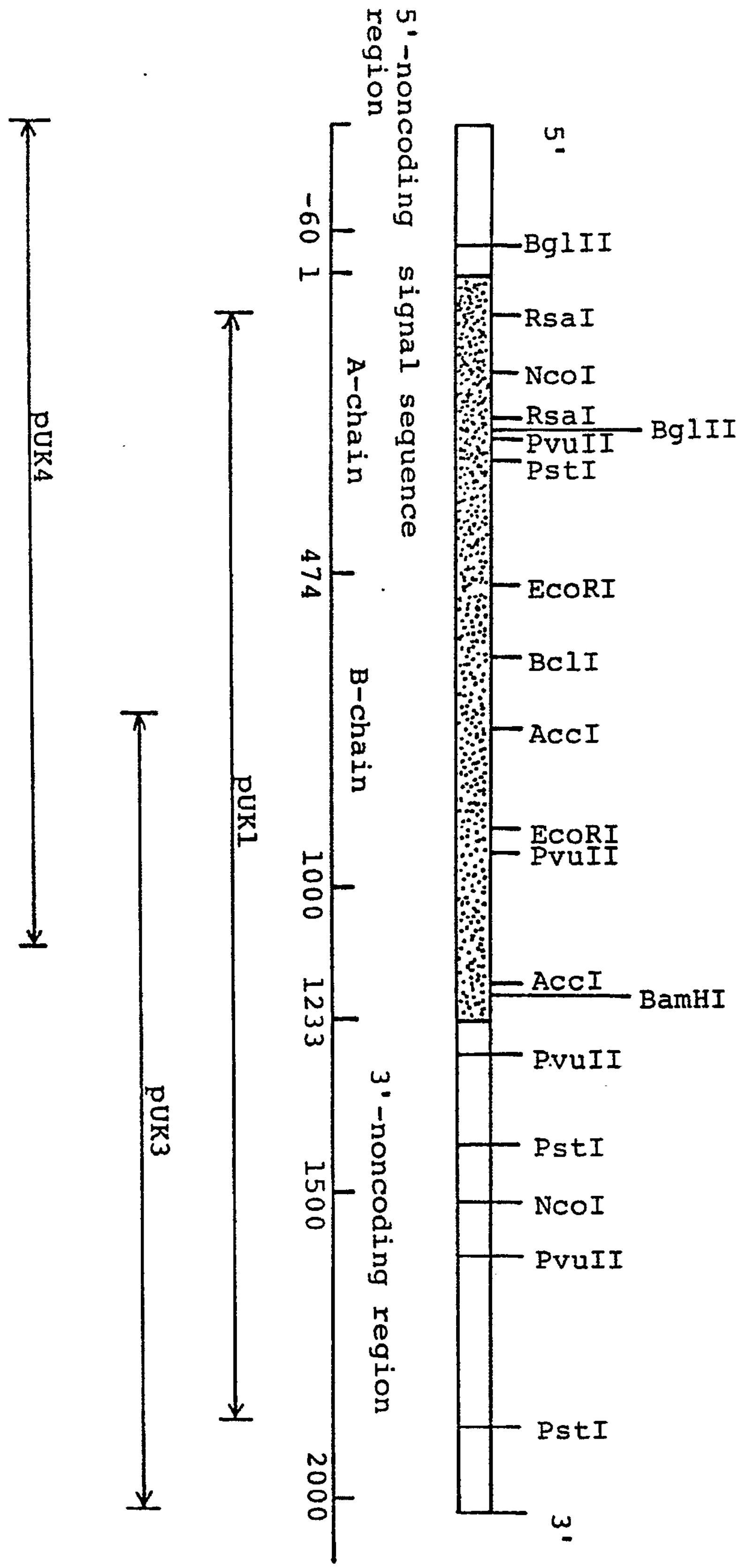

5'
BglII
RsaI
NcoI
RsaI
BglII
PvuII
PstI
EcoRI
BclI
AccI
EcoRI
PvuII
AccI
BamHI
PvuII
PstI
NcoI
PvuII
PstI
3'
5'-noncoding region
signal sequence
A-chain
B-chain
3'-noncoding region
-60
1
474
1000
1233
1500
2000
pUK4
pUK1
pUK3

FIG. 2

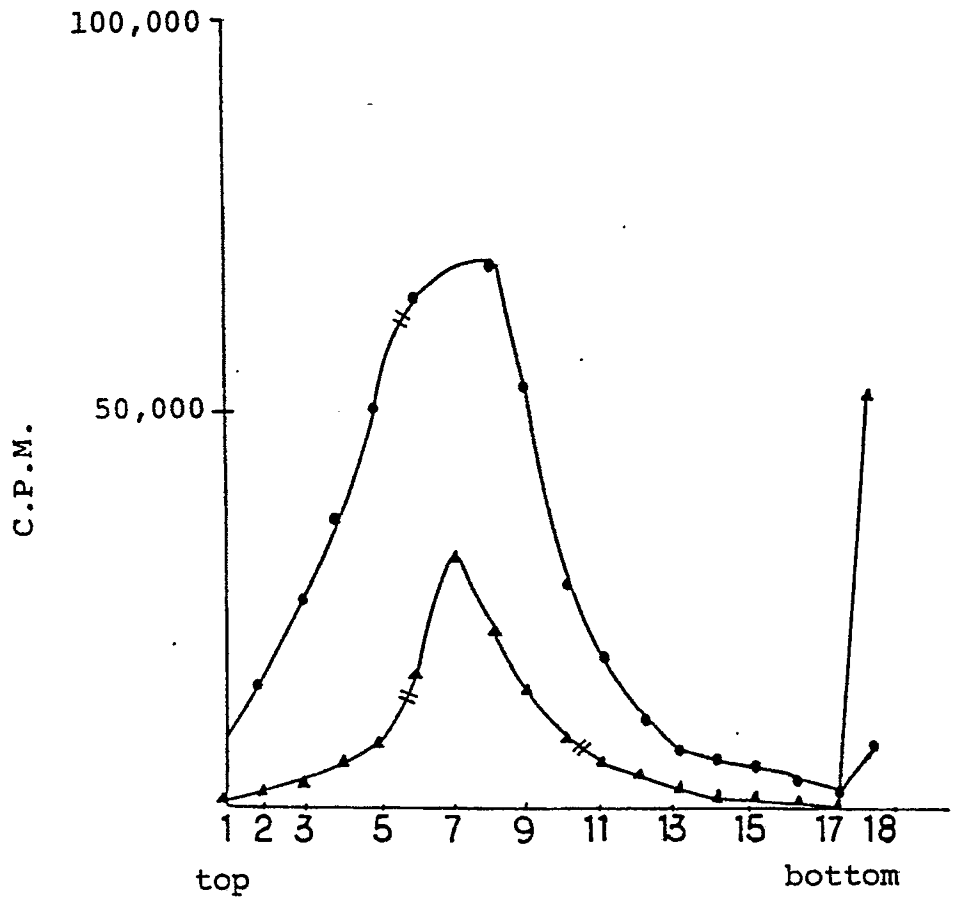

FIG.3

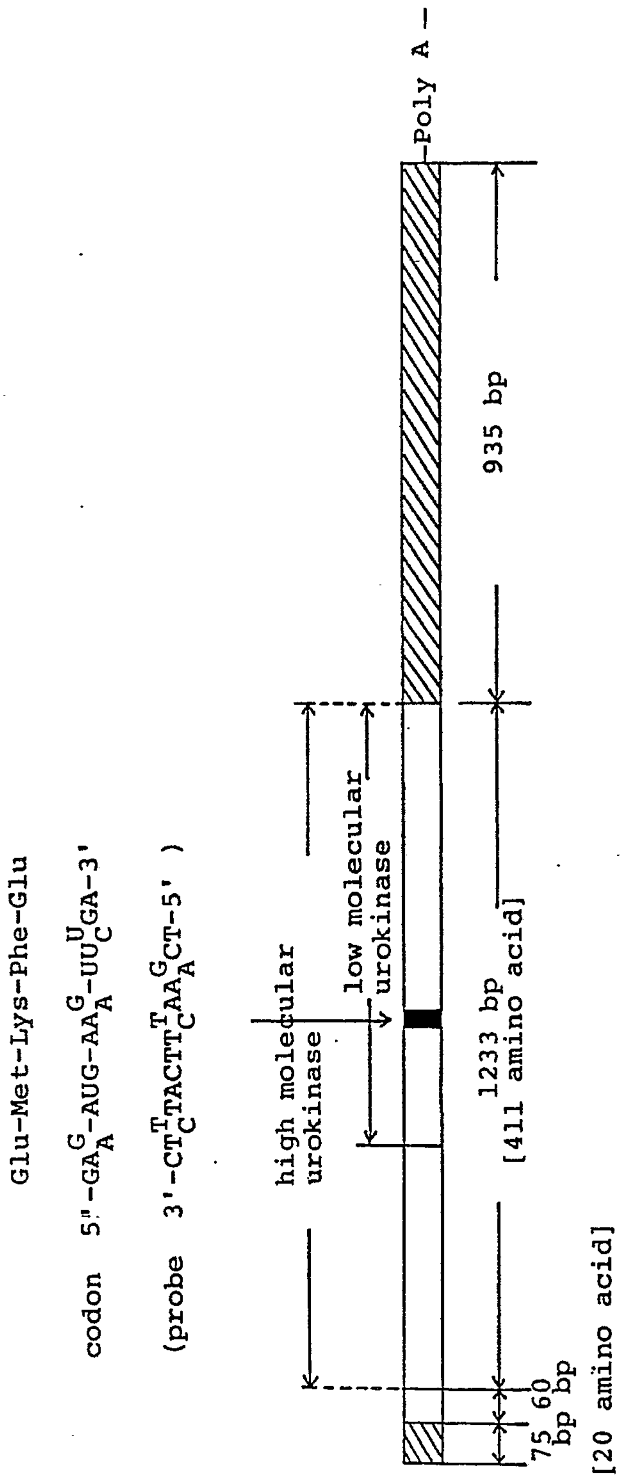
Glu-Met-Lys-Phe-Glu
codon 5'-GA(G/A)-AUG-AA(G/A)-UU(U/C)GA-3'
(probe 3'-CT(T/C)TACTT(T/C)AA(G/A)CT-5')
high molecular urokinase
low molecular urokinase
Poly A
935 bp
1233 bp [411 amino acid]
75 bp
60 bp
[20 amino acid]

FIG.4

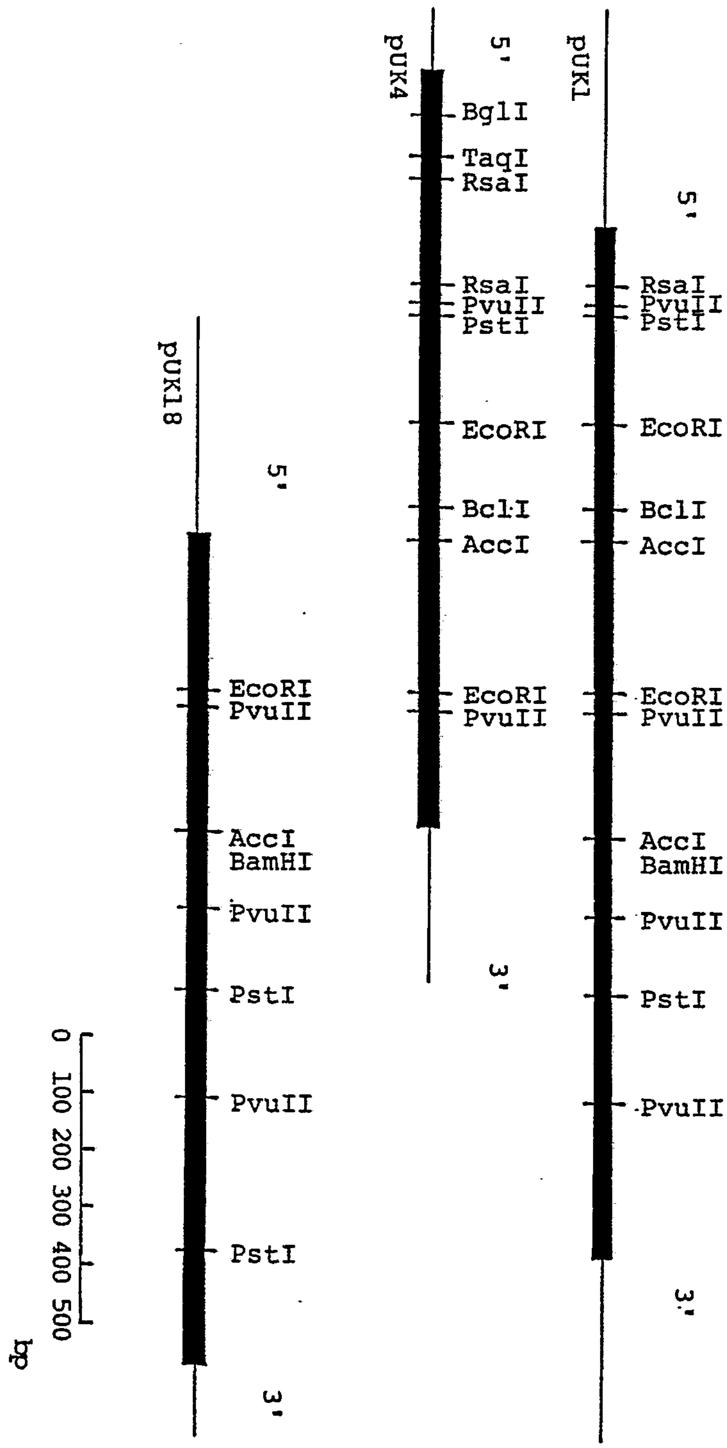
pUK4
5'
BglI
TaqI
RsaI
RsaI
PvuII
PstI
EcoRI
BclI
AccI
EcoRI
PvuII
3'
pUK1
5'
RsaI
PvuII
PstI
EcoRI
BclI
AccI
EcoRI
PvuII
AccI
BamHI
PvuII
PstI
PvuII
3'
pUK18
5'
EcoRI
PvuII
AccI
BamHI
PvuII
PstI
PvuII
PstI
3'
0 100 200 300 400 500
bp

FIG. 5

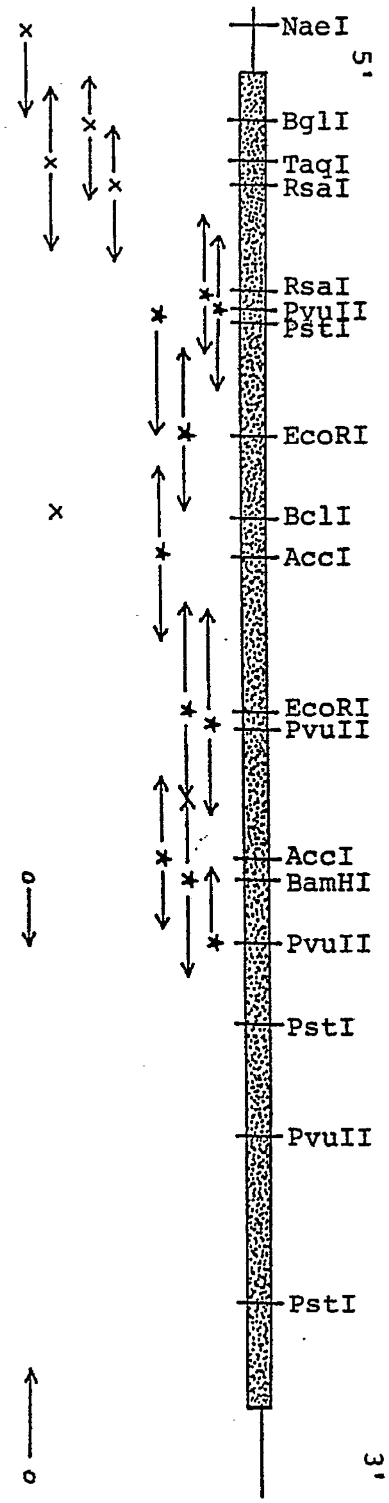
NaeI
5'
BglI
TaqI
RsaI
RsaI
PvuII
PstI
EcoRI
BclI
AccI
EcoRI
PvuII
AccI
BamHI
PvuII
PstI
PvuII
PstI
3'

FIG. 6

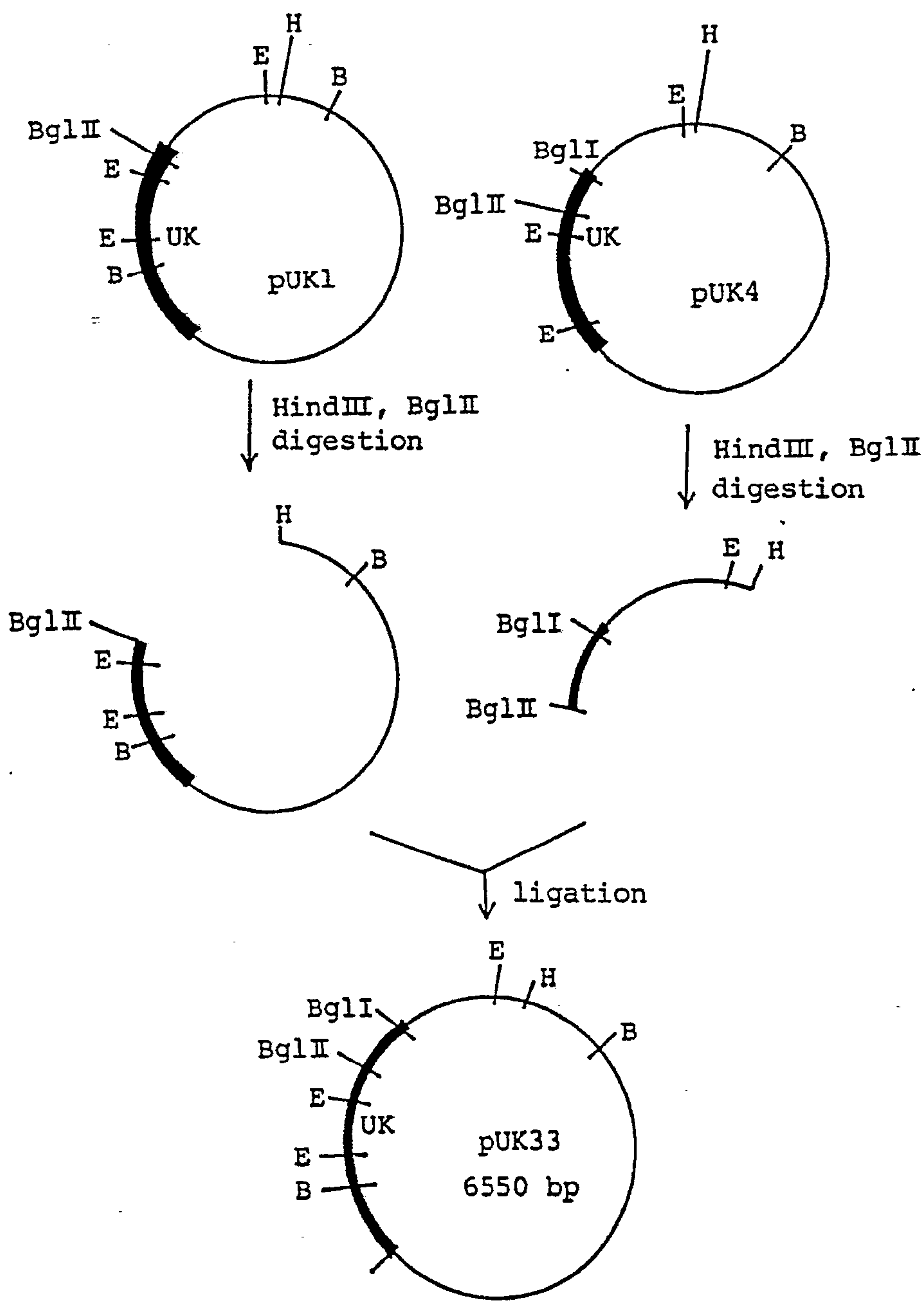
H
E
B
BglII
E
E
UK
B
pUK1
H
E
BglI
B
BglII
E
UK
pUK4
E
HindIII, BglII
digestion
HindIII, BglII
digestion
H
B
BglII
E
E
B
E
H
BglI
BglII
ligation
E
H
BglI
BglII
B
E
UK
E
B
pUK33
6550 bp

FIG. 7

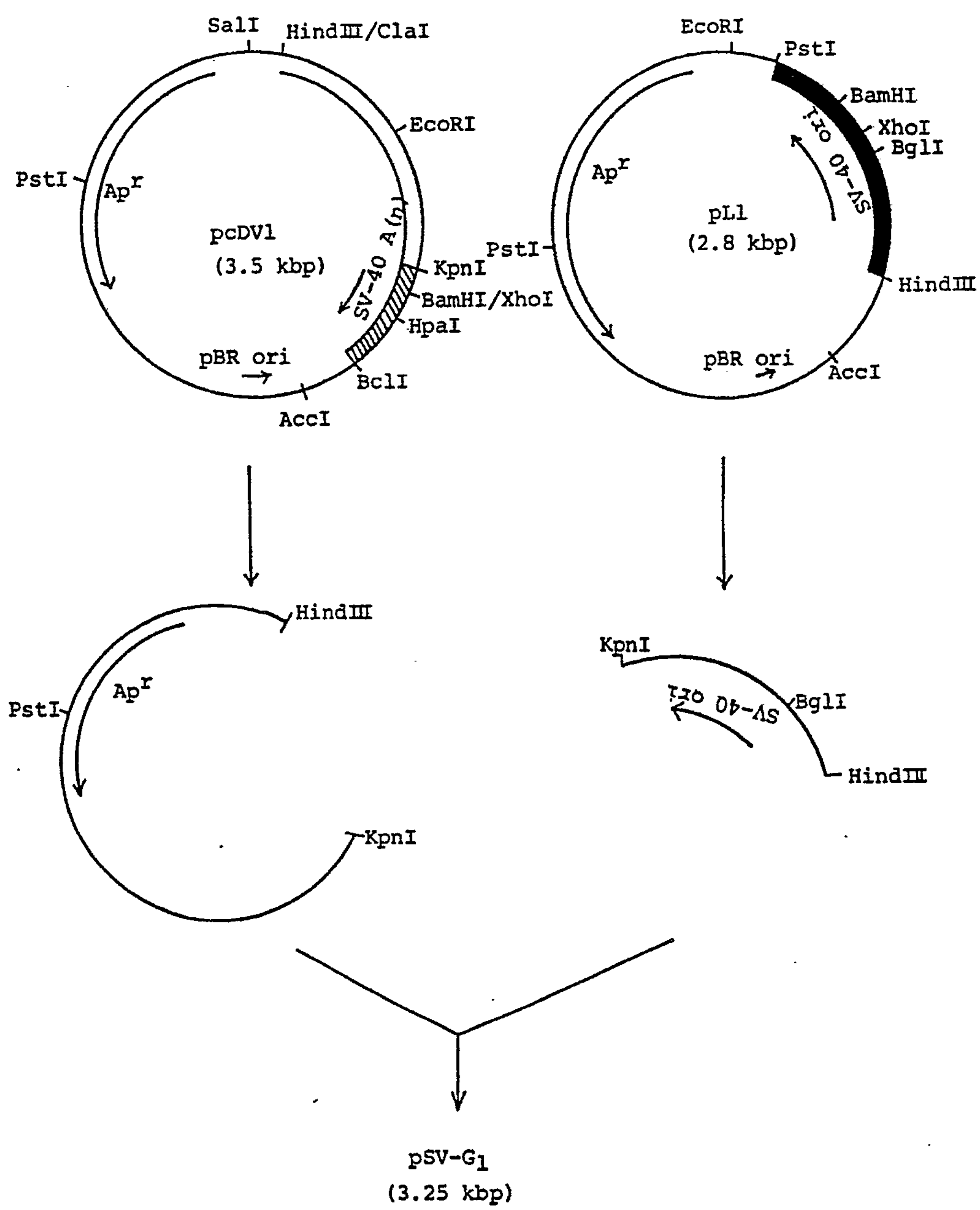
SalI
HindIII/ClaI
EcoRI
PstI
Apr
pcDV1
(3.5 kbp)
SV-40 A(n)
KpnI
BamHI/XhoI
HpaI
BclI
pBR ori
AccI
EcoRI
PstI
BamHI
XhoI
BglI
SV-40 ori
Apr
pL1
(2.8 kbp)
PstI
HindIII
pBR ori
AccI
HindIII
PstI
Apr
KpnI
KpnI
SV-40 ori
BglI
HindIII
pSV-G1
(3.25 kbp)

FIG. 8

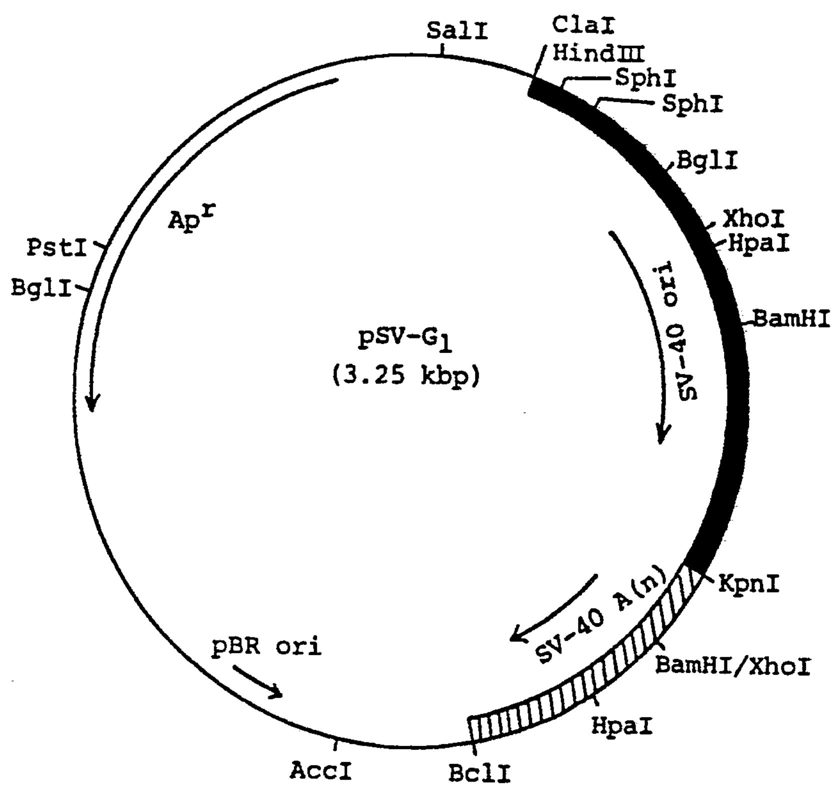
SalI
ClaI
HindIII
SphI
SphI
BglI
XhoI
HpaI
BamHI
KpnI
BamHI/XhoI
HpaI
BclI
AccI
pBR ori
SV-40 A(n)
SV-40 ori
PstI
BglI
Apr
pSV-G1
(3.25 kbp)

FIG. 9

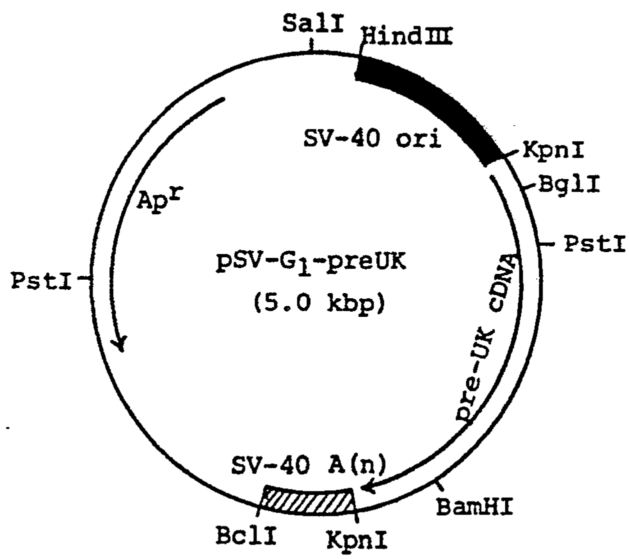
SalI
HindIII
SV-40 ori
KpnI
BglI
PstI
Ap^r
pSV-G1-preUK
(5.0 kbp)
PstI
pre-UK cDNA
SV-40 A(n)
BamHI
BclI
KpnI

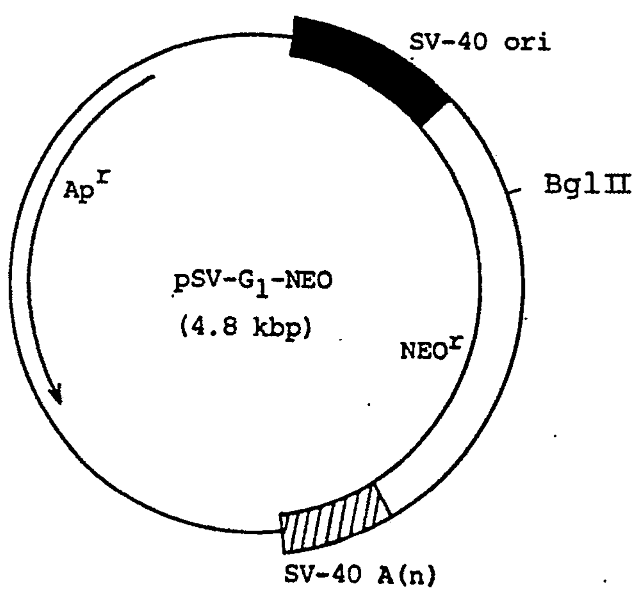
SV-40 ori
Ap^r
BglII
pSV-G1-NEO
(4.8 kbp)
NEO^r
SV-40 A(n)

FIG.10

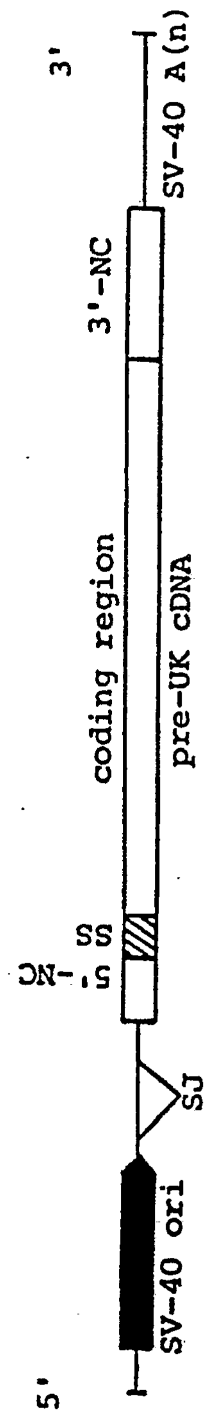

FIG. 11